# EUROPEAN PATENT SPECIFICATION

(11) **EP 0 956 038 B1**
(45) Date of publication and mention of the grant of the patent: **26.03.2003**
(21) Application number: 97948274.2
(22) Date of filing: 14.11.1997
(51) Int. Cl.: A61K 38/18, A61P 27/00

(54) **MORPHOGEN PEPTIDE-INDUCED REGENERATION OF SENSE PERCEPTORY TISSUES**
DIE VON MORPHOGENEN PEPTIDEN HERVORGERUFENE WIEDERHERSTELLUNG VON GEWEBE AUS SINNESZELLEN
REGENERATION INDUITE PAR PEPTIDE MORPHOGENE DE TISSUS PERCEPTO-SENSORIELS

(30) Priority: 15.11.1996 US 751227
(43) Date of publication of application: 17.11.1999
(73) Proprietor: Curis, Inc., Cambridge, MA 02198 (US)
(72) Inventor: SAMPATH, Kuber, Holliston, MA 01746 (US); RUEGER, David, C., Southborough, MA 01772 (US); COHEN, Charles, M., Weston, MA 02139 (US); CHARETTE, Marc, F., Needham, MA 02192 (US); JIN, Donald, F., Shrewsbury, MA 01545 (US)
(74) Representative: Price, Vincent Andrew
(86) International application number: US9720743
(87) International publication number: WO98020890

(56) References cited:
- WO-A-93/14692
- WO-A-94/03200
- WO-A-97/07135
- WO-A-97/17983
- WO-A-97/30722
- WO-A-97/34626

## Description

### Field of the Invention

The invention relates to the use of a morphogen for the manufacture of a medicament for the treatment of disorders of sense organs as defined in claim 1.

### Background of the invention

Mammalian sense organs develop from thickened ectodermal placodes in response to induction by the nervous system. Upon completion of embryogenesis in mammals, each specialized sense organ has only limited repair and regenerative capabilities. Neurodegenerative diseases may result in permanent loss of sensory function. Physical trauma and exposure to toxins adversely affects the function of sense organs. Finally, the aging causes a decrease in the sensitivity of sense organs.

Sensory deficits can take may forms. For example, anosmia, the loss of the sense of smell, may be affected by destruction of the olfactory neuroepithelium, by viral infection, by atrophic or chronic rhinitis, or by neoplasms. Destruction of the olfactory nerves may be caused by head trauma, intracranial surgery, infections, or neoplasms. Asnomia can occur congenitally with male hypogonadism.

Major ophthalmic disorders affecting the eye include macular holes, macular degeneration, retinal detachment, retinal tears, and diabetic retinopathy. Additional retinal disorders include retinal edema, retinal vascular disorders and retinal neovascularization. The most important disorders of the lens of the eye are cataracts and the refractive errors. The most important disorders of the cornea are those related to corneal defects including corneal ulcers and wounds and the consequences of dry eye/Sjögren's syndrome. Additional ophthalmic disorders include wound healing disorders, proliferative disorders, uveitis, secondary cataracts, corneal epithelial wounds, corneal neovascularization, and surgical wounds.

The cornea and conjunctiva are vulnerable to damage from pathogenic agents or direct trauma, drying associated with disorders of tearing, exposure to radiant energy (ultraviolet light, sun and welding guns), allergens such as pollen and mold, and infectious agents. Keratoconjunctivitis can also occur in patients with Stevens-Johnson syndrome, Wegener's granulomatosis, rheumatoid arthritis, atopic dermatitis and cicatricial pemphigoid. Corneal ulcers may also occur. Common types of corneal surgery include cataract extraction, with or without lens replacement; corneal transplants, to treat viral infection or penetrating keratoplasty; glaucoma filtration surgery; and radial keratotomy and other types of surgery to correct refraction.

Hearing loss is among the most common chronic neural impairment in the US population. More that 28 million Americans have impaired hearing varying from a mild but important loss of sensitivity to a total loss of hearing. Hearing loss. including tinnitus, vertigo, earache, and otorrhea, may be characterized as either "conductive" or "sensorineural". Conductive hearing loss is caused by a lesion in the external auditory canal or in the middle ear. Conductive hearing loss is characterized by decreased sensitivity to acoustic stimuli. Examples of conditions leading to conductive hearing loss include otosclerosis, trauma to the tympanic membrane or middle ear, and infections of the middle ear.

A substantial number of hearing impairments are caused by environmental factors in addition to age related hearing loss and genetic disorders. These include acoustic trauma as well as exposure to ototoxic amino glycoside antibiotics. In addition, some antiretroviral and antitumor agents such as Didanosine and Cisplatin are known to be ototoxic.

The inner ear can be divided into two sensory organs: the auditory organ for sound processing and vestibular organ for sensing orientation and motion. Loss of corresponding sensory structures in the vestibular sensory epithelium leads to disequilibrium and vertigo. After the age of 70, there is a loss of up to 40 percent of the sensory hair cells in the semicircular canal cristae and 20 percent of the hair cells in the sacculus and utriculus in the vestibular organs, similar to the age related loss of the sensory hair cells in the auditory organs. Thus, degeneration of the sensory epithlium of either organ can lead to a significant reduction in auditory or vestibular function.

Degeneration of auditory and vestibular function in most cases is due to the loss of the sensory hair cells. Hair cells do not actually have hair but are sensory cells which display sterocilia and kinocilium on the apical surface of the sensory epithelia which are able to transduce mechanical auditory simulation from the outer and middle ear as well as gravity and motion into electrical signals. Attached to these cells are neurons which transmit these signals to the brain. In humans, auditory information is transmitted by approximately 15,000 hair cells in which only about 3,500 form the inner hair cells. The inner hair cells form synapses with approximately 90 percent of the primary auditory neurons; therefore, damage to a small number of these important cells can lead to a significant hearing loss.

Proliferation and regeneration of hair cells has been observed in fish and amphibians. However, it was generally believed that higher vertebrates were not able to regenerate hair cells postnatally. In 1987, it was discovered that birds continually replace hair cells and have a remarkable ability to regenerate damaged hair cells in response to acoustic trauma or ototoxic drugs. For this reason, much of the work on regeneration of vestibular and auditory sensory organs has been done in birds. Efforts have been made to identify growth factors and morphogens that are capable of simulating the proliferation and differentiation of damaged and progenitor cells following an insult. Several growth factors have been found to increase proliferation of the support cells in birds following damage, including FGF, IGF, EGF and TGF-α. Following the observation of regeneration in birds, there has been a significant effort to examine whether hair cells can be regenerated in mammals. So far, no one has been able to show regeneration of hair cells in the auditory sensory epithelium in adult mammals.

Currently no satisfactory therapy exists to regenerate sensory-neural epithelia and repair the damage caused by traumatic injuries and diseases of sense organs such as auditory and vestibular organs.

### Summary of the Invention

The present invention is directed to the use of a morphogen for the manufacture of a medicament for treatment of disorders of sense organs as defined in claim 1. The invention is based, in part, upon the discovery that morphogens restore sensory function, and prevent sensory loss.

A morphogen is to be administered to a patient having a disorder of a sense organ, wherein the morphogen comprises a dimeric protein having an amino acid sequence selected from the group consisting of a sequence have 70% homology with the C-terminal seven-cysteine skeleton of human OP-1 (amino acids 330-341 of SEQ ID NO: 2), a sequence having greater than 60% amino acid sequence identity with human OP-1; generic sequence 7 (SEQ ID NO: 4); generic sequence 8 (SEQ ID NO: 6); generic sequence 10 (SEQ ID NO: 7); and OPX (SEQ ID NO: 3); wherein the morphogen are capable of stimulating production of N-CAM or L1 isoforms by an NG108-15 cell *in vivo*. Sensory perception deficits may result from a disease, degenerative disorder, genetic disorder, mechanical trauma, and chemical trauma. Sensory perception deficits include loss of hearing, olfaction, vision, taste, touch, as well as loss of orientation due to vestibular dysfunction. Administering one of the aforementioned morphogens also provides a prophylactic function. Such administration has the effect of preserving sensory perception in a mammal having, or at risk of having, sensory perception deficits resulting from a disease, degenerative disorder, genetic disorder, mechanical trauma, and chemical trauma.

Specifically for treating (pre- or post-symptomatically) sensory perception deficits a morphogen selected from the group consisting of human OP-1, mouse OP-1, human OP-2, mouse OP-2, 60A, GDF-1, BMP2A, BMP2B, DPP, Vgl, Vgr-1, BMP3, BMP5, and BMP6 can be administered. Such morphogens are capable of stimulating production of N-CAM or L1 isoform by an NG108-15 cell *in vivo*.

In a particularly-preferred embodiment, the morphogen is a soluble complex, comprising at least one morphogen pro domain, or fragment thereof, non-covalently attached to a mature morphogen.

In one aspect of the invention a therapeutically effective amount of a morphogen, as defined herein, is to be administered to a mammal upon sensory perception deficit, or in anticipation of such deficit, for a time and at a concentration sufficient to maintain the integrity of sensory perception tissues, including repairing damaged tissues, regenerating new tissues, or inhibiting additional damage thereto.

In another aspect of the invention a therapeutically effective amount of a morphogen is to be administered to a mammal upon hearing loss, or in anticipation of such hearing loss, for a time and at a concentration sufficient to maintain the integrity of sensory hair cells, including promoting the growth of hair cells, repairing damaged hair cells, regenerating new hair cells, or inhibiting additional damage thereto.

In yet another aspect of the invention a therapeutically effective amount of a morphogen is to be administered to a mammal upon loss or deficit of vestibular function, or in anticipation of such hearing loss or deficit, for a time and at a concentration sufficient to maintain the integrity of sensory hair cells, including promoting the growth of hair cells, or repairing damaged hair cells, regenerating new hair cells, or inhibiting additional damage thereto.

Generally, morphogens useful in the invention are dimeric proteins that induce morphogenesis of one or more eukaryotic (*e.g.*, mammalian) cells, tissues or organs. Tissue morphogenesis includes *de novo* or regenerative tissue formation, such as occurs in a vertebrate embryo during development. Of particular interest are morphogens that induce tissue-specific morphogenesis at least of bone or neural tissue. As defined herein, a morphogen comprises a pair of polypeptides that, when folded, form a dimeric protein that elicits morphogenetic responses in cells and tissues displaying morphogen-specific receptors. That is, the morphogens generally induce a cascade of events including all of the following in a morphogenically permissive environment: stimulating proliferation of progenitor cells; stimulating the differentiation of progenitor cells; stimulating the proliferation of differentiated cells; and, supporting the growth and maintenance of differentiated cells. "Progenitor" cells are uncommitted cells that are competent to differentiate into one or more specific types of differentiated cells, depending on their genomic repertoire and the tissue specificity of the permissive environment in which morphogenesis is induced. An exemplary progenitor cell is a hematopoeitic stem cell, a mesenchymal stem cell, a basement epithelium cell, a neural crest cell, or the like. Further, morphogens can delay or mitigate the onset of senescence- or quiescence-associated loss of phenotype and/or tissue function. Still further, morphogens can stimulate phenotypic expression of a differentiated cell type, including expression of metabolic and/or functional, *e.g.*, secretory, properties thereof. In addition, morphogens can induce redifferentiation of committed cells (*e.g*., osteoblasts, neuroblasts, or the like) under appropriate conditions. As noted above, morphogens that induce proliferation and/or differentiation at least of bone or neural tissue, and/or support the growth, maintenance and/or functional properties of neural tissue, are of particular interest herein. *See*, *e.g*., WO 92/15323, WO 93/04692, WO 94/03200 (providing more detailed disclosures as to the tissue morphogenic properties of these proteins).

As used herein, the terms "morphogen," "bone morphogen," "bone morphogenic protein," "BMP," "morphogenic protein" and "morphogenetic protein" all embrace the class of proteins typified by human osteogenic protein 1 (hOP-1). Nucleotide and amino acid sequences for hOP-1 are provided in SEQ ID NOS: 1 and 2, respectively. For ease of description, hOP-1 is considered a representative morphogen. It will be appreciated that OP-1 is merely representative of the TGF-β subclass of true tissue morphogens and is not intended to limit the description. Other known and useful morphogens include, but are not limited to, BMP-2, BMP-3, BMP-3b, BMP-4, BMP-5, BMP-6, BMP-8, BMP-9, BMP-10, BMP-11, BMP-12, BMP-13, BMP-15, GDF-1, GDF-2, GDF-3, GDF-5, GDF-6, GDF-7, GDF-8, GDF-9, GDF-10, GDF-11, GDF-12, 60A. NODAL, UNIVIN, SCREW, ADMP, and NEURAL, and morphogenically-active amino acid variants of any thereof.

In specific embodiments, useful morphogens include those sharing the conserved seven cysteine skeleton, and sharing at least 70% amino acid sequence homology (similarity), within the C-terminal seven-cysteine skeleton of human OP-1, residues 330-431 of SEQ ID NO: 2 (hereinafter referred to as the "reference sequence"). In another embodiment, the invention encompasses use of biologically active species (phylogenetic) variants of any of the morphogenic proteins recited herein, including conservative amino acid sequence variants, proteins encoded by degenerate nucleotide sequence variants, and morphogenically-active proteins sharing the conserved seven cysteine skeleton as defined herein and encoded by a DNA competent to hybridize under standard stringency conditions to a DNA encoding a morphogenic protein disclosed herein, including, without limitation, OP-1 or BMP-2 or BMP-4. Presently, however, the reference sequence is that of residues 330-431 of SEQ ID NO: 2 (OP-1).

In still another embodiment, morphogens useful in the invention are defined as morphogenically-active proteins having any one of the generic sequences defined herein, including OPX (SEQ ID NO: 3) and Generic Sequences 7 and 8 (SEQ ID NOS: 4 and 5, respectively), or Generic Sequences 9 and 10 (SEQ ID NOS: 6 and 7, respectively). OPX encompasses the observed variation between the known phylogenetic counterparts of the osteogenic OP-1 and OP-2 proteins, and is described by the amino acid sequence presented herein below and in SEQ ID NO: 3. Generic Sequence 9 is a 96 amino acid sequence containing the C-terminal six cysteine skeleton observed in hOP-1 (residues 335-431 of SEQ ID NO: 2) and wherein the remaining residues encompass the observed variation among OP-1, OP-2, OP-3, BMP-2, BMP-3, BMP-4, BMP-5, BMP-6, BMP-8, BMP-9, BMP-10, BMP-11, BMP-15, GDF-1, GDF-3, GDF-5, GDF-6, GDF-7, GDF-8, GDF-9, GDF-10, GDF-11, 60A, UNIVIN, NODAL, DORSALIN, NEURAL, SCREW and ADMP. That is, each of the non-cysteine residues is independently selected from the corresponding residue in this recited group of known, naturally-sourced proteins. Generic Sequence 10 is a 102 amino acid sequence which includes a five amino acid sequence added to the N-terminus of the Generic Sequence 9 and defines the seven cysteine skeleton observed in hOP-1 (330-431 SEQ ID NO: 2). Generic Sequences 7 and 8 are 96 and 102 amino acid sequences, respectively, containing either the six cysteine skeleton (Generic Sequence 7) or the seven cysteine skeleton (Generic Sequence 8) defined by hOP-1 and wherein the remaining non-cysteine residues encompass the observed variation among OP-1, OP-2, OP-3, BMP-2, BMP-3, BMP-4, 60A, DPP, Vgl, BMP-5, BMP-6, Vgr-1, and GDF-1.

Of particular interest are morphogens which, when provided to a specific tissue of a mammal, induce tissue-specific morphogenesis or maintain the normal state of differentiation and growth of that tissue. In preferred embodiments, the present morphogens induce the formation of vertebrate (*e.g*., avian or mammalian) body tissues, such as but not limited to nerve, eye, bone, cartilage, bone marrow, ligament, tooth dentin, periodontium, liver, kidney, lung, heart, or gastrointestinal lining. Preferred methods may be carried out in the context of developing embryonic tissue, or at an aseptic, unscarred wound site in post-embryonic tissue. Methods of identifying such morphogens, or morphogen receptor agonists, are known in the art and include assays for compounds which induce morphogen-mediated responses (*e.g.*, induction of endochondral bone formation, induction of differentiation of metanephric mesenchyme, and the like). In a preferred embodiment, morphogens of the invention, when implanted in a mammal in conjunction with a matrix permissive of bone morphogenesis, are capable of inducing a developmental cascade of cellular and molecular events that culminates in endochondral bone formation. *See,* U.S. 4,968,590; Sampath, *et al*., *Proc. Natl. Acad. Sci. USA 80*: 6591-6595 (1983).

In an alternative preferred embodiment, morphogens used according to the invention are also capable of stimulating production of cell adhesion molecules, including nerve cell adhesion molecules (N-CAMs). In a preferred embodiment, the present morphogens are capable of stimulating the production of N-CAM *in vitro* in NG108-15 cells, which are a preferred model system for assessing neuronal differentiation, particularly motor neuron differentiation.

In still other embodiments, an agent which acts as an agonist of a morphogen receptor may be administered instead of the morphogen itself. An "agonist" of a receptor is a compound which binds to the receptor, and for which the result of such binding is similar to the result of binding the natural, endogenous ligand of the receptor. That is, the compound must, upon interaction with the receptor, produce the same or substantially similar transmembrane and/or intracellular effects as the endogenous ligand. Thus, an agonist of a morphogen receptor binds to the receptor and such binding has the same or a functionally similar result as morphogen binding (*e.g*., induction of morphogenesis). The activity or potency of an agonist can be less than that of the natural ligand, in which case the agonist is said to be a "partial agonist," or it can be equal to or greater than that of the natural ligand, in which case it is said to be a "full agonist." Thus, for example, a small peptide or other molecule which can mimic the activity of a morphogen in binding to and activating the morphogen's receptor may be employed as an equivalent of the morphogen. Preferably the agonist is a full agonist, but partial morphogen receptor agonists may also be advantageously employed. Such an agonist may also be referred to as a morphogen "mimic," "mimetic," or "analog."

Morphogen inducers and agonists can be identified by mutation, site-specific mutagenesis, combinatorial chemistry, etc. Such methods are well known in the art. For example, methods of identifying morphogen inducers or agonists of morphogen receptors may be found in U.S.S.N. 08/478,097 filed June 7, 1995 and U.S.S.N. 08/507,598 filed July 26, 1995. Candidate morphogen inducers and agonists are then tested for their ability to induce endochondral bone formation and preferably, to stimulate N-CAM production in neurons or in a neuronal model system, such as NG108-15 cells. Morphogen inducers and agonists identified according to the present invention are capable of inducing endochondral bone formation when implanted in a mammal in conjunction with a matrix permissive of bone morphogenesis and are capable of stimulating production of N-CAM *in vitro.*

WO 97/34626, to be considered persuant to Art. 54(3) EPC, discloses treatment by OP-1 of a sensory perception deficit in hearing, vision, touch, taste, proprioception, and olfaction following an ischemic or traumatic injury.

### Brief Description of the Figures

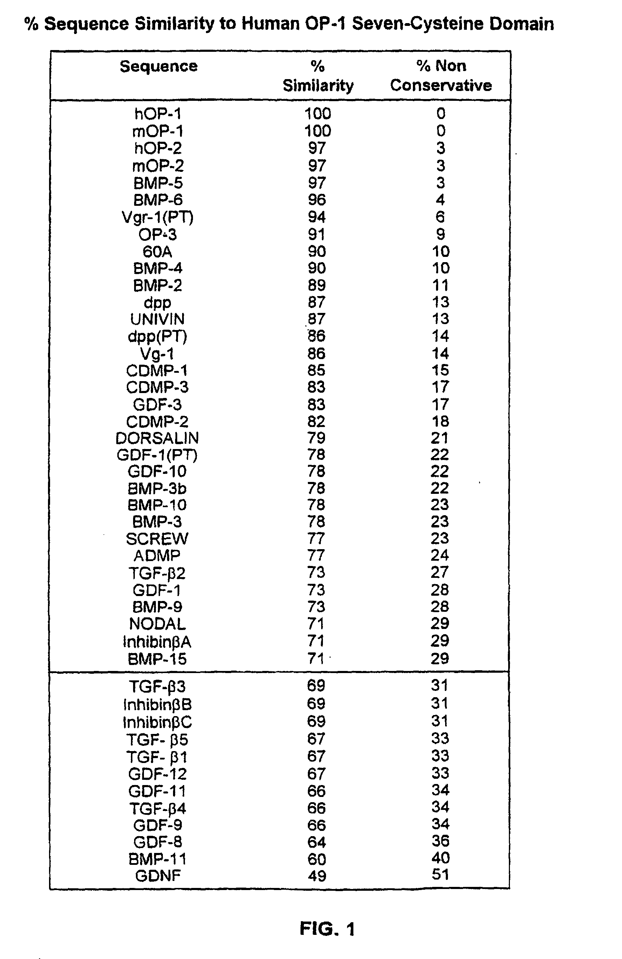

FIG. 1 is a tabular presentation of the percent amino acid sequence identity and percent amino acid sequence homology ("similarity") that various members of the family of morphogenic proteins as defined herein share with hOP-1 in the C-terminal seven cysteine skeleton.

### Detailed Description of the Invention

### I. Definitions

In order to more clearly and concisely point out the subject matter of the claimed invention, the following definitions are provided for specific terms used in the following written description and appended claims.

"Ophthalmic tissue" refers to the tissues which comprise the structure of the eye, including, but not limited to, the optic nerve, retina, vitreous humor, lens, cornea, sclera, macula, and conjunctiva.

"Ophthalmic disorder" refers to physiologic abnormalities of the eye. They may involve the retina, the vitreous humor, lens, cornea, sclera or other portions of the eye, or physiologic abnormalities which adversely affect the eye, such as inadequate tear production.

"Retinal wounds" include, but are not limited to, tears and holes in the retina and separation from the underlying choroid. Retinal wounds can appear, for example, after trauma, cystic degeneration, vitreoretinal traction, myopic degeneration, laser photocoagulation, lightning strike, pilocarpine administration and cataract extraction.

"Macular degeneration" is characterized by the excessive buildup of fibrous deposits in the macula and retina and the atrophy of the retinal pigment epithelium.

"Secondary cataracts" are opacities in the ocular lens which interfere with vision. Secondary cataracts can occur, for example, after x-ray exposure, in diabetes, Wilson's disease and galactosemia, and as side effects in drug therapy.

The term "diseased corneal tissue" includes damage to the cornea by a variety of causes including, but not limited to, trauma, dry eyes (in which the conjunctiva on the inside of the eyelid may abrade the cornea), excessive light, allergens and infectious agents.

"Sjögren's syndrome" is an autoimmune disorder which frequently is characterized by dry eyes, due to autoimmune destruction of the tear glands.

"Ocular neovascularization" or "ophthalmic neovascularization" is herein defined as the unwanted new growth of blood vessels into the ocular (ophthalmic) tissues. If unchecked, such growth can result in blindness. The ocular tissues which can be invaded by neovascularization include, for example, the cornea, iris, retina, vitreous, and choroid. Many diseases and conditions cause or contribute to ocular neovascularization. Causes of corneal neovascularization include but are not limited to trauma, chemical burns or corneal transplantation. Causes of neovascularization of the iris include but are not limited to diabetic retinopathy, vein occlusion, ocular tumor and retinal detachment. Causes of retinal and intravitreal neovascularization include but are not limited to diabetic retinopathy, vein occlusion, sickle cell retinopathy, retinopathy of prematurity, retinal detachment, ocular ischemia and trauma. Causes of choroidal neovascularization include but are not limited to retinal disorders of age-related macular degeneration, presumed ocular histoplasmosis syndrome, myopic degeneration, angioid streaks and ocular trauma.

"Treating a mammal for ocular trauma neovascularization" is herein defined as treating ocular neovascularization which has already become detectable.

Provided below are detailed descriptions of suitable morphogens useful in this invention, as well as methods for their administration and application, and numerous, nonlimiting examples which (1) illustrate the suitability of the morphogens, morphogen inducers and morphogen receptor agonists described herein as therapeutic agents for maintaining function of sense perception organs, particularly eyes, ears and nose; and (2) provide assays with which to test candidate morphogens, inducers and analogs for their efficacy in the methods and compositions disclosed herein.

As used herein, a subject is said to be afflicted with a disorder of a sense organ if the subject has any detectable (*e.g.*, clinically significant) impairment of function of the eye, ear or nose. Whether a particular subject is at risk of developing a disorder of a sense organ is a determination which may routinely be made by one of ordinary skill in the relevant medical or veterinary art.

### II. Description of the Preferred Embodiments

### A. Biochemical, Structural and Functional Properties of Useful Morphogenic Proteins

As noted above, a protein is morphogenic as defined herein if it induces the developmental cascade of cellular and molecular events that culminate in the formation of new, organ-specific tissue. In a preferred embodiment, a morphogen is a dimeric protein, each polypeptide component of which has a sequence that corresponds to, or is functionally equivalent to, at least the conserved C-terminal six or seven cysteine skeleton of human OP-1, included in SEQ ID NO: 2, and/or which shares 70% amino acid sequence homology with OP-1 in this region. The morphogens are generally competent to induce a cascade of events including the following, in a morphogenically permissive environment: stimulating proliferation of progenitor cells; stimulating the differentiation of progenitor cells; stimulating the proliferation of differentiated cells; and supporting the growth and maintenance of differentiated cells. Under appropriate conditions morphogens are also competent to induce redifferentiation of cells that have undergone abnormal differentiation. Details of how the morphogens useful in this invention were identified, as well as a description on how to make, use and test them for morphogenic activity are disclosed in numerous publications, including U.S. Patent Nos. 5,011,691 and 5,266,683, and the international patent application publications WO 92/15323; WO 93/04692; and WO 94/03200. As disclosed therein, the morphogens can be purified from naturally-sourced material or recombinantly produced from prokaryotic or eukaryotic host cells, using the genetic sequences disclosed therein. Alternatively, novel morphogenic sequences can be identified following the procedures disclosed therein.

The naturally-occurring morphogens share substantial amino acid sequence homology in their C-terminal sequences (sharing *e.g.*, a six or seven cysteine skeleton sequence). Typically, a naturally-occurring morphogen is translated as a precursor, having an N-terminal signal peptide sequence, typically less than about 35 residues in length, followed by a "pro" domain that is cleaved to yield the mature polypeptide, which includes the biologically active C-terminal skeleton sequence. The signal peptide is cleaved rapidly upon translation, at a cleavage site that can be predicted in a given sequence using the method of Von Heijne, *Nucleic Acids Research 14*: 4683-4691 (1986). The pro polypeptide typically is about three times larger than the fully processed, mature C-terminal polypeptide. Under native conditions, the protein is secreted as a mature dimer and the cleaved pro polypeptide is thought to remain associated therewith to form a protein complex, presumably to improve the solubility of the mature dimeric protein. The complexed form of a morphogen is generally observed to be more soluble than the mature form under physiological conditions.

Natural-sourced morphogenic protein in its mature, native form, is typically a glycosylated dimer, having an apparent molecular weight of about 30-36 kDa as determined by SDS-PAGE. When reduced, the 30 kDa protein gives rise to two glycosylated polypeptide subunits having apparent molecular weights in the range of about 16 kDa and about 18 kDa. The unglycosylated dimeric protein, which also has morphogenic activity, typically has an apparent molecular weight in the range of about 27 kDa. When reduced, the 27 kDa protein gives rise to two unglycosylated polypeptides having molecular weights typically in the range of about 14 kDa to about 16 kDa.

In preferred embodiments, each of the polypeptide subunits of a dimeric morphogenic protein as defined herein comprises an amino acid sequence sharing a defined relationship with an amino acid sequence of a reference morphogen. In one embodiment, preferred morphogenic polypeptide chains share a defined relationship with a sequence present in morphogenically-active human OP-1, SEQ ID NO: 2. However, any one or more of the naturally-occurring or biosynthetic morphogenic proteins disclosed herein similarly could be used as a reference sequence. Preferred morphogenic polypeptide chains share a defined relationship with at least the C-terminal six cysteine skeleton of human OP-1, residues 335-431 of SEQ ID NO: 2. Preferably, morphogenic proteins share a defined relationship with at least the C-terminal seven cysteine skeleton of human OP-1, residues 330-431 of SEQ ID NO: 2.

Functionally equivalent sequences include functionally equivalent arrangements of cysteine residues disposed within the reference sequence, including amino acid insertions or deletions which alter the linear arrangement of these cysteines, but do not materially impair their relationship in the folded structure of the dimeric morphogen protein, including their ability to form such intra- or inter-chain disulfide bonds as may be necessary for morphogenic activity. For example naturally-occurring morphogens have been described in which at least one internal deletion (of one residue; BMP2) or insertion (of four residues; GDF-1) is present but does not abrogate biological activity. Functionally equivalent sequences further include those wherein one or more amino acid residues differ from the corresponding residue of a reference sequence, *e.g*., the C-terminal seven cysteine skeleton of human OP-1, provided that this difference does not destroy tissue morphogenic activity. Accordingly, conservative substitutions of corresponding amino acids in the reference sequence are preferred. Amino acid residues that are "conservative substitutions" for corresponding residues in a reference sequence are those that are physically or functionally similar to the corresponding reference residues, *e.g.*, that have similar size, shape, electric charge, chemical properties including the ability to form covalent or hydrogen bonds, or the like. Particularly preferred conservative substitutions are those fulfilling the criteria defined for an accepted point mutation in Dayhoff, *et al.*, 5 ATLAS OF PROTEIN SEQUENCE AND STRUCTURE, Suppl. 3, ch. 22 pp. 354-352 (1978), Natl. Biomed. Res. Found., Washington, D.C. 20007, the teachings of which are incorporated by reference herein. Examples of conservative substitutions include the substitution of one amino acid for another with similar characteristics, *e.g*., substitutions within the following groups: valine, glycine; glycine, alanine; valine, isoleucine, leucine; aspartic acid, glutamic acid; asparagine, glutamine; serine, threonine; lysine, arginine; and phenylalanine, tyrosine. The term "conservative substitution" also includes the use of a synthetic or derivatized amino acid in place of the corresponding natural parent amino acid, provided that antibodies raised to the resulting variant polypeptide also immunoreact with the corresponding naturally sourced morphogen polypeptide.

The following publications disclose publications morphogen polypeptide sequences, as well as relevant chemical and physical properties, of naturally-occurring and/or synthetic morphogens: OP-1 and OP-2: U.S. 5,011,691, U.S. 5,266,683, Ozkaynak, *et al., EMBO J. 9*: 2085-2093 (1990); OP-3: WO 94/10203 (PCT US93/10520); *BMP-2, BMP-3,* and BMP-4: WO 88/00205, Wozney, *et al., Science 242*: 1528-1534 (1988); BMP-5 and BMP-6: Celeste, *et al., PNAS 87*: 9843-9847 (1991); Vgr-1: Lyons, *et al., PNAS 86*: 4554-4558 (1989); DPP: Padgett, *et al., Nature 325*: 81-84 (1987); Vg-1: Weeks *Cell 51:* 861-867 (1987); BMP-9: WO 95/33830 (PCT/US95/07084); BMP-10: WO 94/26893 (PCT/US94/05290); BMP-11: WO 94/26892 (PCT/US94/05288); BMP-12: WO 95/16035 (PCT/US94/14030); BMP-13: WO 95/16035 (PCT/US94/14030); GDF-1: WO 92/00382 (PCT/US91/04096) and Lee, *et al.*, *PNAS 88*: 4250-4254 (1991); GDF-8: WO 94/21681 (PCT/US94/03019); GDF-9: WO-94/-15966-(PCT/US94/00685); GDF-10: WO 95/10539 (PCT/US94/11440); GDF-11: WO 96/01845 (PCT/US95/08543); BMP-15: WO 96/36710 (PCT/US96/06540); *MP121:* WO 96/01316 (PCT/EP95/02552); GDF-5 (CDMP-1, MP52): WO 94/15949 (PCT/US94/00657) and WO 96/14335 (PCT/US94/12814) and WO 93/16099 (PCT/EP93/00350); GDF-6 (CDMP-2, BMP-13): WO 95/01801 (PCT/US94/07762) and WO 96/14335 and WO 95/10635 (PCT/US94/14030); GDF-7 (CDMP-3, BMP-12): WO 95/10802 (PCT/US94/07799) and WO 95/10635 (PCT/US94/14030). In another embodiment, useful proteins include biologically active biosynthetic constructs, including novel biosynthetic morphogenic proteins and chimeric proteins designed using sequences from two or more known morphogens. See also the biosynthetic constructs disclosed in U.S. Pat. 5,011,691 (*e.g*., COP-1, COP-3, COP-4, COP-5, COP-7, and COP-16).

In certain preferred embodiments, useful morphogenic proteins include those in which the amino acid sequences comprise a sequence sharing at least 70% amino acid sequence homology or "similarity", and preferably 80% homology or similarity, with a reference morphogenic protein selected from the exemplary, naturally-occurring morphogenic proteins listed herein. Preferably, the reference protein is human OP-1, and the reference sequence thereof is the C-terminal seven cysteine skeleton present in osteogenically active forms of human OP-1, residues 330-431 of SEQ ID NO: 2. Useful morphogenic proteins accordingly include allelic, phylogenetic counterpart and other variants of the preferred reference sequence, whether naturally-occurring or biosynthetically produced (*e.g.*, including "muteins" or "mutant proteins"), as well as novel members of the general morphogenic family of proteins including those set forth and identified above. Certain particularly preferred morphogenic polypeptides share at least 60% amino acid identity with the preferred reference sequence of human OP-1, still more preferably at least 65% amino acid identity therewith.

In certain embodiments, a polypeptide suspected of being functionally equivalent to a reference morphogen polypeptide is aligned therewith using the method of Needleman, *et al., J. Mol. Biol. 48:* 443-453 (1970), implemented conveniently by computer programs such as the Align program (DNAstar, Inc.). As noted above, internal gaps and amino acid insertions in the candidate sequence are ignored for purposes of calculating the defined relationship, conventionally expressed as a level of amino acid sequence homology, or identity, between the candidate and reference sequences. "Amino acid sequence homology" is understood herein to include both amino acid sequence identity and similarity. Homologous sequences share identical and/or similar amino acid residues, where similar residues are conservation substitutions for, or "allowed point mutations" of, corresponding amino acid residues in an aligned reference sequence. Thus, a candidate polypeptide sequence that shares 70% amino acid homology with a reference sequence is one in which any 70% of the aligned residues are either identical to, or are conservative substitutions of, the corresponding residues in a reference sequence. In a preferred embodiment, the reference sequence is the C-terminal seven cysteine skeleton sequence of human OP-1.

FIG. 1 recites the percent amino acid sequence homology (similarity) and percent identity within the C-terminal seven cysteine skeletons of various representative members of the TGF-β family, using OP-I as the reference sequence. The percent homologies recited in the figure are determined by aligning the sequences essentially following the method of Needleman, *et al., J. Mol. Biol., 48*: 443-453 (1970), and using the Align Program (DNAstar, Inc.). Insertions and deletions from the reference morphogen sequence (the C-terminal, biologically active seven-cysteine skeleton of hOP-1) are ignored for purposes of calculation.

As is apparent to one of ordinary skill in the art reviewing the sequences for the proteins listed in FIG. 1, significant amino acid changes can be made from the reference sequence while retaining substantial morphogenic activity. For example, while the GDF-1 protein sequence shares only about 50% amino acid identity with the hOP-1 sequence described herein, the GDF-1 sequence shares greater than 70% amino acid sequence homology with the hOP-1 sequence, where "homology" is as defined above. Moreover, GDF-1 contains a four amino acid insert (Gly-Gly-Pro-Pro) between the two residues corresponding to residue 372 and 373 of OP-1 (SEQ ID NO: 2). Similarly, BMP-3 has a "extra" residue, a valine, inserted between the two residues corresponding to residues 385 and 386 of hOP-1 (SEQ ID NO: 2). Also, BMP-2 and BMP-4 are both "missing" the amino acid residue corresponding to residue 389 of OP-1 (SEQ ID NO: 2). None of these "deviations" from the reference sequence appear to interfere substantially with biological activity.

In other preferred embodiments, the family of morphogenic polypeptides useful in the present invention, and members thereof, are defined by a generic amino acid sequence. For example, Generic Sequence 7 (SEQ ID NO: 4) and Generic Sequence 8 (SEQ ID NO: 5) disclosed below, encompass the observed variations between preferred protein family members identified to date, including at least OP-1, OP-2, OP-3, CBMP-2A, CBMP-2B, BMP-3, 60A, DPP, Vg1, BMP-5, BMP-6, Vgr-1, and GDF-1. The amino acid sequences for these proteins are described herein and/or in the art. as summarized above. The generic sequences include both the amino acid identity shared by these sequences in the C-terminal skeleton, defined by the six and seven cysteine skeletons (Generic Sequences 7 and 8, respectively), as well as alternative residues for the variable positions within the sequence. The generic sequences provide an appropriate cysteine skeleton where inter- or intramolecular disulfide bonds can form, and contain certain critical amino acids likely to influence the tertiary structure of the folded proteins. In addition, the generic sequences allow for an additional cysteine at position 36 (Generic Sequence 7) or position 41 (Generic Sequence 8), thereby encompassing the morphogenically-active sequences of OP-2 and OP-3.

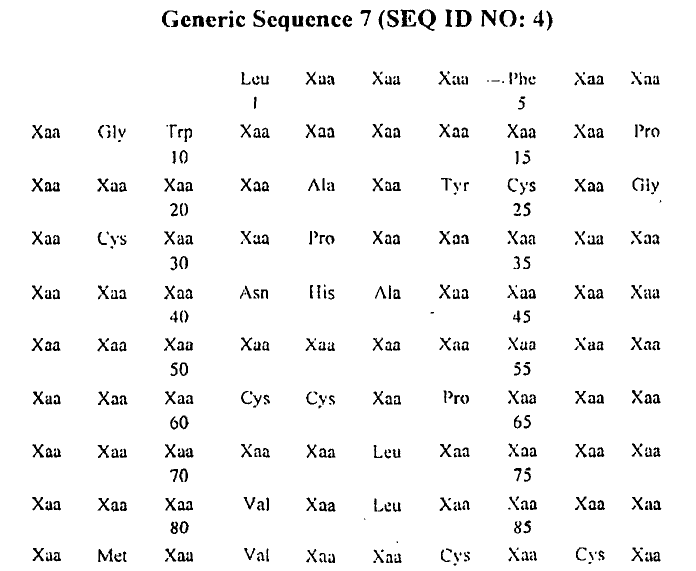

wherein each Xaa independently is selected from a group of one or more specified amino acids defined as follows: "Res. " means "residue" and Xaa at res. 2 = (Tyr or Lys); Xaa at res. 3 = Val or Ile); Xaa at res. 4 = (Ser, Asp or Glu); Xaa at res. 6 = (Arg, Gln, Ser, Lys or Ala); Xaa at res. 7 = (Asp or Glu); Xaa at res. 8 = (Leu, Val or Ile); Xaa at res. 11 = (Gln, Leu, Asp, His, Asn or Ser): Xaa at res. 12 = (Asp, Arg, Asn or Glu); Xaa at res. 13 = (Trp or Ser); Xaa at res. 14 = (Ile or Val); Xaa at res. 15 = (Ile or Val); Xaa at res. 16 (Ala or Ser); Xaa at res. 18 = (Glu, Gin, Leu, Lys, Pro or Arg); Xaa at res. 19 = (Gly or Ser); Xaa at res. 20 = (Tyr or Phe); Xaa at res. 21 = (Ala, Ser, Asp, Met, His, Gln, Leu or Gly); Xaa at res. 23 = (Tyr, Asn or Phe); Xaa at res. 26 = (Glu, His, Tyr, Asp, Gln, Ala or Ser); Xaa at res. 28 = (Glu, Lys, Asp, Gln or Ala); Xaa at res. 30 = (Ala, Ser, Pro, Gln, Ile or Asn); Xaa at res. 31 = (Phe, Leu or Tyr); Xaa at res. 33 = (Leu, Val or Met); Xaa at res. 34 = (Asn, Asp, Ala, Thr or Pro); Xaa at res. 35 = (Ser, Asp, Glu, Leu, Ala or Lys); Xaa at res. 36 = (Tyr, Cys, His, Ser or Ile); Xaa at res. 37 = (Met, Phe, Gly or Leu); Xaa at res. 38 = (Asn, Ser or Lys): Xaa at res. 39 = (Ala, Ser, Gly or Pro); Xaa at res. 40 = (Thr, Leu or Ser); Xaa at res. 44 = (Ile, Val or Thr); Xaa at res. 45 = (Val, Leu, Met or Ile); Xaa at res. 46 = (Gln or Arg); Xaa at res. 47 = (Thr, Ala or Ser); Xaa at res. 48 = (Leu or Ile); Xaa at res. 49 = (Val or Met); Xaa at res. 50 = (His, Asn or Arg); Xaa at res. 51 = (Phe, Leu, Asn, Ser, Ala or Val); Xaa at res. 52 = (Ile, Met, Asn, Ala, Val, Gly or Leu); Xaa at res. 53 = (Asn, Lys, Ala, Glu, Gly or Phe); Xaa at res. 54 = (Pro, Ser or Val); Xaa at res. 55 = (Glu, Asp, Asn, Gly, Val, Pro or Lys); Xaa at res. 56 = (Thr, Ala, Val, Lys, Asp, Tyr, Ser, Gly, Ile or His); Xaa at res. 57 = (Val, Ala or Ile); Xaa at res. 58 = (Pro or Asp); Xaa at res. 59 = (Lys, Leu or Glu); Xaa at res. 60 = (Pro, Val or Ala); Xaa at res. 63 = (Ala or Val); Xaa at res. 65 = (Thr, Ala or Glu); Xaa at res. 66 = (Gln, Lys, Arg or Glu); Xaa at res. 67 = (Leu, Met or Val); Xaa at res. 68 = (Asn, Ser, Asp or Gly); Xaa at res. 69 = (Ala, Pro or Ser); Xaa at res. 70 = (Ile, Thr, Val or Leu); Xaa at res. 71 = (Ser, Ala or Pro): Xaa at res. 72 = (Val, Leu, Met or Ile); Xaa at res. 74 = (Tyr or Phe); Xaa at res. 75 = (Phe, Tyr, Leu or His); Xaa at res. 76 = (Asp, Asn or Leu); Xaa at res. 77 = (Asp, Glu, Asn, Arg or Ser); Xaa at res. 78 = (Ser, Gln, Asn, Tyr or Asp); Xaa at res. 79 = (Ser, Asn, Asp, Glu or Lys); Xaa at res. 80 = (Asn, Thr or Lys); Xaa at res. 82 = (Ile, Val or Asn); Xaa at res. 84 = (Lys or Arg); Xaa at res. 85 = (Lys, Asn, Gln, His, Arg or Val); Xaa at res. 86 = (Tyr, Glu or His); Xaa at res. 87 = (Arg, Gln, Glu or Pro); Xaa at res. 88 = (Asn, Glu, Trp or Asp); Xaa at res. 90 = (Val, Thr, Ala or Ile); Xaa at res. 92 = (Arg, Lys, Val, Asp, Gln or Glu); Xaa at res. 93 = (Ala, Gly, Glu or Ser); Xaa at res. 95 = (Gly or Ala) and Xaa at res. 97 = (His or Arg).

Generic Sequence 8 (SEQ ID NO: 5) includes all of Generic Sequence 7 (SEQ ID NO: 4) and in addition includes the following sequence (SEQ ID NO: 8) at its N-terminus: Accordingly, beginning with residue 7, each "Xaa" in Generic Sequence 8 is a specified amino acid defined as for Generic Sequence 7, with the distinction that each residue number described for Generic Sequence 7 is shifted by five in Generic Sequence 8. Thus, "Xaa at res. 2 =(Tyr or Lys)" in Generic Sequence 7 refers to Xaa at res. 7 in Generic Sequence 8. In Generic Sequence 8, Xaa at res. 2 = (Lys, Arg, Ala or Gln); Xaa at res. 3 = (Lys, Arg or Met); Xaa at res. 4 = (His, Arg or GIn); and Xaa at res. 5 = (Glu, Ser, His, Gly, Arg, Pro, Thr, or Tyr).

In another embodiment, useful osteogenic proteins include those defined by Generic Sequences 9 and 10 (SEQ ID NOS: 6 and 7, respectively), described herein above. Specifically, Generic Sequences 9 and 10 are composite amino acid sequences of the following proteins: human OP-1, human OP-2, human OP-3, human BMP-2, human BMP-3, human BMP-4, human BMP-5, human BMP-6, human BMP-8, human BMP-9, human BMP-10, human BMP-11, *Drosophila* 60A, Xenopus Vg-1, sea urchin UNIVIN, human CDMP-1 (mouse GDF-5), human CDMP-2 (mouse GDF-6, human BMP-13), human CDMP-3 (mouse GDF-7, human BMP-12), mouse GDF-3. human GDF-1, mouse GDF-1, chicken DORSALIN, *Drosophila* dpp, *Drosophila* SCREW, mouse NODAL, mouse GDF-8, human GDF-8, mouse GDF-9, mouse GDF-10, human GDF-11, mouse GDF-11, human BMP-15, and rat BMP-3b. Like Generic Sequence 7. Generic Sequence 9 accommodates the C-terminal six cysteine skeleton and, like Generic Sequence 8, Generic Sequence 10 accommodates the seven cysteine skeleton.

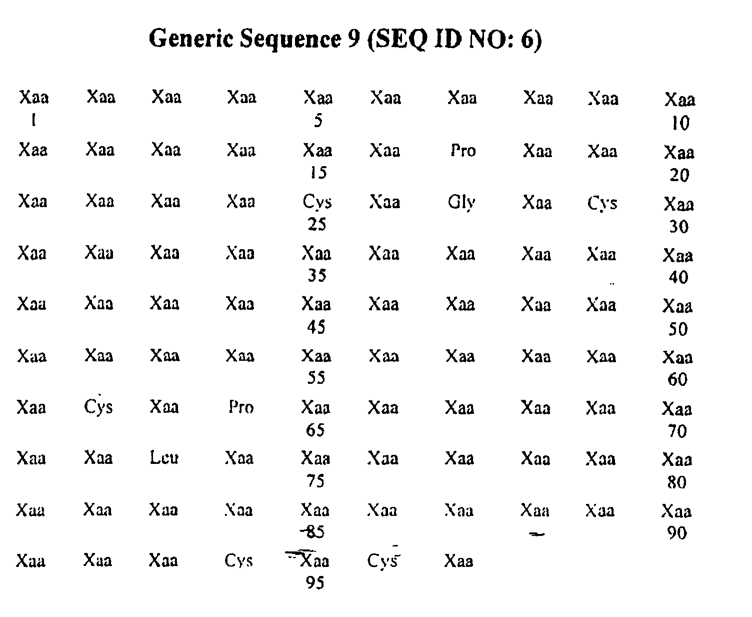

wherein each Xaa is independently selected from a group of one or more specified amino acids defined as follows: "Res. " means "residue" and Xaa at res. 1 = (Phe, Leu or Glu); Xaa at res. 2 = (Tyr, Phe, His, Arg, Thr, Lys, Gln, Val or Glu); Xaa at res. 3 = (Val, Ile, Leu or Asp); Xaa at res. 4 = (Ser, Asp, Glu, Asn or Phe); Xaa at res. 5 = (Phe or Glu); Xaa at res. 6 = (Arg, Gln, Lys, Ser, Glu, Ala or Asn); Xaa at res. 7 = (Asp, Glu, Leu, Ala or Gln); Xaa at res. 8 = (Leu, Val, Met, Ile or Phe); Xaa at res. 9 = (Gly, His or Lys); Xaa at res. 10 = (Trp or Met); Xaa at res. 11 = (Gln, Leu, His, Glu, Asn, Asp, Ser or Gly); Xaa at res. 12 = (Asp, Asn, Ser, Lys, Arg, Glu or His); Xaa at res. 13 = (Trp or Ser); Xaa at res. 14 = (Ile or Val); Xaa at res. 15 = (Ile or Val); Xaa at res. 16 = (Ala, Ser, Tyr or Trp); Xaa at res. 18 = (Glu, Lys, Gln, Met, Pro, Leu, Arg, His or Lys); Xaa at res. 19 = (Gly, Glu, Asp, Lys, Ser, Gln, Arg or Phe); Xaa at res. 20 = (Tyr or Phe); Xaa at res. 21 = (Ala, Ser, Gly, Met, Gln, His, Glu, Asp, Leu, Asn, Lys or Thr); Xaa at res. 22 = (Ala or Pro); Xaa at res. 23 = (Tyr, Phe, Asn, Ala or Arg); Xaa at res. 24 = (Tyr, His, Glu, Phe or Arg); Xaa at res. 26 = (Glu, Asp, Ala, Ser, Tyr, His, Lys, Arg, Gin or Gly); Xaa at res. 28 = (Glu, Asp, Leu, Val, Lys, Gly, Thr, Ala or Gln); Xaa at res. 30 = (Ala, Ser, Ile, Asn, Pro, Glu, Asp, Phe, Gin or Leu); Xaa at res. 31 = (Phe, Tyr, Leu, Asn, Gly or Arg); Xaa at res. 32 = (Pro, Ser, Ala or Val); Xaa at res. 33 = (Leu, Met, Glu, Phe or Val); Xaa at res. 34 = (Asn, Asp, Thr, Gly, Ala, Arg, Leu or Pro); Xaa at res. 35 = (Ser, Ala, Glu, Asp, Thr, Leu, Lys, Gln or His); Xaa at res. 36 = (Tyr, His, Cys, Ile, Arg, Asp, Asn, Lys, Ser, Glu or Gly); Xaa at res. 37 = (Met, Leu, Phe, Val, Gly or Tyr); Xaa at res. 38 = (Asn, Glu, Thr, Pro, Lys, His, Gly, Met, Val or Arg); Xaa at res. 39 = (Ala, Ser, Gly, Pro or Phe); Xaa at res. 40 = (Thr, Ser, Leu, Pro, His or Met); Xaa at res. 41 = (Asn, Lys, Val, Thr or Gln); Xaa at res. 42 = (His, Tyr or Lys); Xaa at res. 43 = (Ala, Thr, Leu or Tyr); Xaa at res. 44 = (Ile, Thr, Val, Phe, Tyr, Met or Pro); Xaa at res. 45 = (Val, Leu, Met, Ile or His); Xaa at res. 46 = (Gln, Arg or Thr); Xaa at res. 47 = (Thr, Ser, Ala, Asn or His); Xaa at res. 48 = (Leu, Asn or Ile); Xaa at res. 49 = (Val, Met, Leu, Pro or Ile); Xaa at res. 50 = (His, Asn. Arg, Lys. Tyr or Gln); Xaa at res. 51 = (Phe, Leu, Ser, Asn, Met, Ala, Arg, Glu, Gly or Gln); Xaa at res. 52 = (Ile, Met, Leu, Val, Lys, Gln, Ala or Tyr); Xaa at res. 53 = (Asn, Phe, Lys, Glu, Asp, Ala, Gln, Gly, Leu or Val); Xaa at res. 54 = (Pro, Asn, Ser, Val or Asp); Xaa at res. 55 = (Glu, Asp, Asn, Lys, Arg, Ser, Gly, Thr, Gln, Pro or His); Xaa at res. 56 = (Thr, His, Tyr, Ala, Ile, Lys, Asp, Ser, Gly or Arg); Xaa at res. 57 = (Val, Ile, Thr, Ala, Leu or Ser); Xaa at res. 58 = (Pro, Gly, Ser, Asp or Ala); Xaa at res. 59 = (Lys, Leu, Pro, Ala, Ser, Glu, Arg or Gly); Xaa at res. 60 = (Pro, Ala, Val, Thr or Ser); Xaa at res. 61 = (Cys, Val or Ser); Xaa at res. 63 = (Ala, Val or Thr); Xaa at res. 65 = (Thr, Ala, Glu, Val, Gly, Asp or Tyr); Xaa at res. 66 = (Gln, Lys, Glu, Arg or Val); Xaa at res. 67 = (Leu, Met, Thr or Tyr); Xaa at res. 68 = (Asn, Ser, Gly, Thr, Asp, Glu, Lys or Val); Xaa at res. 69 = (Ala, Pro, Gly or Ser); Xaa at res. 70 = (Ile, Thr, Leu or Val); Xaa at res. 71 = (Ser, Pro, Ala, Thr, Asn or Gly); Xaa at res. 2 = (Val, Ile, Leu or Met); Xaa at res. 74 = (Tyr, Phe, Arg, Thr, Tyr or Met); Xaa at res. 75 = (Phe, Tyr, His, Leu, Ile, Lys, Gln or Val); Xaa at res. 76 = (Asp, Leu, Asn or Glu); Xaa at res. 77 = (Asp, Ser, Arg, Asn, Glu, Ala, Lys, Gly or Pro); Xaa at res. 78 = (Ser, Asn, Asp, Tyr, Ala, Gly, Gln, Met, Glu, Asn or Lys); Xaa at res. 79 = (Ser, Asn, Glu, Asp, Val, Lys, Gly, Gln or Arg); Xaa at res. 80 = (Asn, Lys, Thr, Pro, Val, Ile, Arg, Ser or Gln); Xaa at res. 81 = (Val, Ile, Thr or Ala): Xaa at res. 82 = (Ile, Asn, Val, Leu, Tyr, Asp or Ala); Xaa at res. 83 = (Leu, Tyr, Lys or Ile); Xaa at res, 84 = (Lys, Arg, Asn, Tyr, Phe, Thr, Glu or Gly); Xaa at res. 85 = (Lys, Arg, His, Gln, Asn, Glu or Val); Xaa at res. 86 = (Tyr, His, Glu or Ile); Xaa at res. 87 = (Arg, Glu, Gln, Pro or Lys); Xaa at res. 88 = (Asn, Asp, Ala, Glu, Gly or Lys); Xaa at res. 89 = (Met or Ala); Xaa at res. 90 = (Val, Ile, Ala, Thr, Ser or Lys); Xaa at res. 91 = (Val or Ala); Xaa at res. 92 = (Arg, Lys, Gln, Asp, Glu, Val, Ala, Ser or Thr); Xaa at res. 93 = (Ala, Ser, Glu, Gly, Arg or Thr); Xaa at res. 95 = (Gly, Ala or Thr); Xaa at res. 97 = (His, Arg, Gly, Leu or Ser). Further, after res. 53 in rBMP-3b and mGDF-10 there is an Ile; after res. 54 in GDF-1 there is a T; after res. 54 in BMP-3 there is a V; after res. 78 in BMP-8 and Dorsalin there is a G; after res. 37 in hGDF-1 there is Pro, Gly, Gly, Pro.

Generic Sequence 10 (SEQ ID NO: 7) includes all of Generic Sequence 9 (SEQ ID NO: 6) and in addition includes the following sequence (SEQ ID NO: 9) at its N-terminus: Accordingly, beginning with residue 6, each "Xaa" in Generic Sequence 10 is a specified amino acid defined as for Generic Sequence 9, with the distinction that each residue number described for Generic Sequence 9 is shifted by five in Generic Sequence 10. Thus, "Xaa at res. 1 =( Tyr, Phe, His, Arg, Thr, Lys, Gln, Val or Glu)" in Generic Sequence 9 refers to Xaa at res. 6 in Generic Sequence 10. In Generic Sequence 10, Xaa at res. 2 = (Lys, Arg, Gln, Ser, His, Glu, Ala, or Cys); Xaa at res. 3 = (Lys, Arg, Met, Lys, Thr, Leu, Tyr, or Ala); Xaa at res. 4 = (His, Gln, Arg, Lys, Thr, Leu, Val, Pro, or Tyr); and Xaa at res. 5 = (Gln, Thr, His, Arg, Pro, Ser, Ala, Gln, Asn, Tyr, Lys, Asp, or Leu).

Based upon alignment of the naturally-occurring morphogens within the definition of Generic Sequence 10, it should be clear that gaps and/or insertions of one or more amino acid residues can be tolerated (without abrogating or substantially impairing biological activity) at least between or involving residues 11-12, 42-43, 59-60, 68-69 and 83-84.

As noted above, certain preferred morphogenic polypeptide sequences useful in this invention have greater than 60% identity, preferably greater than 65% identity, with the amino acid sequence defining the preferred reference sequence of hOP-1. These particularly preferred sequences include allelic and phylogenetic counterpart variants of the OP-1 and OP-2 proteins, including the *Drosophila* 60A protein, as well as the closely related proteins BMP-5, BMP-6 and Vgr-1. Accordingly, in certain particularly preferred embodiments, useful morphogenic proteins include active proteins comprising pairs of polypeptide chains within the generic amino acid sequence herein referred to as "OPX" (SEQ ID NO: 3), which defines the seven cysteine skeleton and accommodates the homologies between several identified variants of OP-1 and OP-2. Accordingly, each "Xaa" at a given position in OPX independently is selected from the residues occurring at the corresponding position in the C-terminal sequence of mouse or human OP-1 or OP-2. Specifically, each "Xaa" is independently selected from a group of one or more specified amino acids as defined below: wherein Xaa at res. 2 = (Lys or Arg); Xaa at res. 3 = (Lys or Arg); Xaa at res. 11 = (Arg or Gin); Xaa at res. 16 = (Gln or Leu); Xaa at res. 19 = (Ile or Val); Xaa at res. 23 = (Glu or Gln); Xaa at res. 26 = (Ala or Ser); Xaa at res. 35 = (Ala or Ser); Xaa at res. 39 = (Asn or Asp); Xaa at res. 41 = (Tyr or Cys); Xaa at res. 50 = (Val or Leu); Xaa at res. 52 = (Ser or Thr); Xaa at res. 56 = (Phe or Leu); Xaa at res. 57 = (Ile or Met); Xaa at res. 58 = (Asn or Lys); Xaa at res. 60 = (Glu, Asp or Asn); Xaa at res. 61 = (Thr, Ala or Val); Xaa at res. 65 = (Pro or Ala); Xaa at res. 71 = (Gln or Lys); Xaa at res. 73 = (Asn or Ser); Xaa at res. 75 = (Ile or Thr); Xaa at res. 80 = (Phe or Tyr); Xaa at res. 82 = (Asp or Ser); Xaa at res. 84 = (Ser or Asn); Xaa at res. 89 = (Lys or Arg); Xaa at res. 91 = (Tyr or His); and Xaa at res. 97 = (Arg or Lys).

In still another preferred embodiment, useful morphogenically-active proteins have polypeptide chains with amino acid sequences comprising a sequence encoded by a nucleic acid that hybridizes with DNA or RNA encoding reference morphogen sequences, *e.g.*, C-terminal sequences defining the conserved seven cysteine skeletons of OP-1, OP-2, BMP-2, BMP-4, BMP-5, BMP-6, 60A, GDF-3, GDF-5, GDF-6, GDF-7 and the like. As used herein, high stringency hybridization conditions are defined as hybridization according to known techniques in 40% formamide, 5 X SSPE, 5 X Denhardt's Solution, and 0.1% SDS at 37°C overnight, and washing in 0.1 X SSPE, 0.1% SDS at 50°C. Standard stringency conditions are well characterized in standard molecular biology cloning texts. See, for example, MOLECULAR CLONING A LABORATORY MANUAL, 2nd Ed., ed. by Sambrook, Fritsch and Maniatis (Cold Spring Harbor Laboratory Press: 1989); DNA CLONING, Volumes I and II (D.N. Glover ed., 1985); OLIGONUCLEOTIDE SYNTHESIS (M.J. Gait ed., 1984); NUCLEIC ACID HYBRIDIZATION (B. D. Hames & S.J. Higgins eds. 1984); and B. Perbal, A PRACTICAL GUIDE TO MOLECULAR CLONING (1984).

In other embodiments, as an alternative to the administration of a morphogenic protein, an effective amount of an agent competent to stimulate or induce increased endogenous morphogen expression in a mammal may be administered by any of the routes described herein. Such a morphogen inducer may be provided to a mammal, *e.g*., by systemic administration to the mammal or by direct administration to the neural tissue. A method for identifying and testing inducers (stimulating agents) competent to modulate the levels of endogenous morphogens in a given tissue is described in published applications WO93/05172 and WO93/05751. Briefly, candidate compounds are identified and tested by incubation *in vitro* with test tissue or cells, or a cultured cell line derived therefrom, for a time sufficient to allow the compound to affect the production, *i.e*., cause the expression and/or secretion, of a morphogen produced by the cells of that tissue. Suitable tissue, or cultured cells of a suitable tissue, are preferably selected from renal epithelium, ovarian tissue, fibroblasts, and osteoblasts.

In yet other embodiments, an agent which acts as an agonist of a morphogen receptor may be administered instead of the morphogen itself. Such an agent may also be referred to an a morphogen "mimic,'' "mimetic," or "analog." Thus, for example, a small peptide or other molecule which can mimic the activity of a morphogen in binding to and activating the morphogen's receptor may be employed as an equivalent of the morphogen. Preferably the agonist is a full agonist, but partial morphogen receptor agonists may also be advantageously employed. Methods of identifying such agonists are known in the art and include assays for compounds which induce morphogen-mediated responses (*e.g*., induction of differentiation of metanephric mesenchyme, induction of endochondral bone formation). For example, methods of identifying morphogen inducers or agonists of morphogen receptors may be found in U.S.S.N. 08/478,097 filed June 7, 1995 and U.S.S.N. 08/507,598 filed July 26, 1995.

As a general matter, medicaments manufactured according to the present invention may be applied to the treatment of any mammalian subject at risk of or afflicted with a neural tissue insult or neuropathy. The invention is suitable for the treatment of any primate, preferably a higher primate such as a human. In addition, however, the invention may be employed in the treatment of domesticated mammals which are maintained as human companions (*e.g*., dogs, cats, horses), which have significant commercial value *(e.g.,* goats, pigs, sheep, cattle, sporting or draft animals), which have significant scientific value (*e.g.*, captive or free specimens of endangered species, or inbred or engineered animal strains), or which otherwise have value.

### C. Formulations and Methods of Treatment

Morphogens, morphogen inducers, or agonists of morphogen receptors of the present invention may be administered by any route which is compatible with the particular morphogen, inducer, or agonist employed. Thus, as appropriate, administration may be oral or parenteral, including intravenous and intraperitoneal routes of administration. In addition, administration may be by periodic injections of a bolus of the morphogen, inducer or agonist, or may be made more continuous by intravenous or intraperitoneal administration from a reservoir which is external (*e.g.*, an i.v. bag) or internal (*e.g.*, a bioerodable implant, or a colony of implanted, morphogen-producing cells).

Therapeutic agents of the invention (*i.e*., morphogens, morphogen inducers or agonists of morphogen receptors) may be provided to an individual by any suitable means, directly (*e.g*., locally, as by injection, implantation or topical administration to a tissue locus) or systemically (*e.g.*, parenterally or orally). Where the agent is to be provided parenterally, such as by intravenous, subcutaneous, intramolecular, ophthalmic, intraperitoneal, intramuscular, buccal, rectal, vaginal, intraorbital, intracerebral, intracranial, intraspinal, intraventricular, intrathecal, intracisternal, intracapsular, intranasal or by aerosol administration, the agent preferably comprises part of an aqueous or physiologically compatible fluid suspension or solution. Thus, the morphogen carrier or vehicle is physiologically acceptable so that in addition to delivery of the desired agent to the patient, it does not otherwise adversely affect the patient's electrolyte and/or volume balance. The fluid medium for the agent thus can comprise normal physiologic saline (*e.g*., 9.85% aqueous NaCl, 0.15M, pH 7-7.4).

Association of the mature morphogen dimer with a morphogen pro domain results in the pro form of the morphogen which typically is more soluble in physiological solutions than the corresponding mature form. In fact, endogenous morphogens are thought to be transported (*e.g*., secreted and circulated) in the mammalian body in this form. This soluble form of the protein can be obtained from culture medium of morphogen-secreting mammalian cells, *e.g*., cells transfected with nucleic acid encoding and competent to express the morphogen. Alternatively, a soluble species can be formulated by complexing the mature, morphogenically-active polypeptide dimer (or an active fragment thereof) with a morphogen pro domain polypeptide or a solubility-enhancing fragment thereof. Solubility-enhancing pro domain fragments can be any N-terminal, C-terminal or internal fragment of the pro region of a member of the morphogen family that complexes with the mature polypeptide dimer to enhance stability and/or dissolubility of the resulting noncovalent or convalent complex. Typically, useful fragments are those cleaved at the proteolytic site Arg-Xaa-Xaa-Arg. A detailed description of soluble complex forms of morphogenic proteins, including how to make, test and use them, is described in WO 94/03600 (PCT US 93/07189). In the case of OP-1, useful pro domain polypeptide fragments include the intact pro domain polypeptide (residues 30-292) and fragments 48-292 and 158-292, all of SEQ ID NO: 2. Another molecule capable of enhancing solubility and particularly useful for oral administrations, is casein. For example, addition of 0.2% casein increases solubility of the mature active form of OP-1 by 80%. Other components found in milk and/or various serum proteins may also be useful.

Useful solutions for parenteral administration may be prepared by any of the methods well known in the pharmaceutical art. described, for example, in REMINGTON'S PHARMACEUTICAL SCIENCES (Gennaro, A., ed.), Mack Pub., 1990. Formulations of the therapeutic agents of the invention may include, for example, polyalkylene glycols such as polyethylene glycol, oils of vegetable origin, hydrogenated naphthalenes, and the like. Formulations for direct administration, in particular, may include glycerol and other compositions of high viscosity to help maintain the agent at the desired locus. Biocompatible, preferably bioresorbable, polymers, including, for example, hyaluronic acid, collagen, tricalcium phosphate, polybutyrate, lactide, and glycolide polymers and lactide/glycolide copolymers, may be useful excipients to control the release of the agent *in vivo.* Other potentially useful parenteral delivery systems for these agents include ethylene-vinyl acetate copolymer particles, osmotic pumps, implantable infusion systems, and liposomes. Formulations for inhalation administration contain as excipients, for example, lactose, or may be aqueous solutions containing, for example, polyoxyethylene-9-lauryl ether, glycocholate and deoxycholate, or oily solutions for administration in the form of nasal drops, or as a gel to be applied intranasally. Formulations for parenteral administration may also include glycocholate for buccal administration, methoxysalicylate for rectal administration, or cutric acid for vaginal administration. Suppositories for rectal administration may also be prepared by mixing the morphogen, inducer or agonist with a non-irritating excipient such as cocoa butter or other compositions which are solid at room temperature and liquid at body temperatures.

Formulations for topical administration to the skin surface may be prepared by dispersing the morphogen, inducer or agonist with a dermatologically acceptable carrier such as a lotion, cream, ointment or soap. Particularly useful are carriers capable of forming a film or layer over the skin to localize application and inhibit removal. For topical administration to internal tissue surfaces, the agent may be dispersed in a liquid tissue adhesive or other substance known to enhance adsorption to a tissue surface. For example, hydroxypropylcellulose or fibrinogen/thrombin solutions may be used to advantage. Alternatively, tissue-coating solutions, such as pectin-containing formulations may be used.

Alternatively, the agents described herein may be administered orally. Oral administration of proteins as therapeutics generally is not practiced, as most proteins are readily degraded by digestive enzymes and acids in the mammalian digestive system before they can be absorbed into the bloodstream. However, the morphogens described herein typically are acid stable and protease-resistant (see, for example, U.S. Pat. No. 4,968,590). In addition, at least one morphogen, OP-1, has been identified in mammary gland extract. colostrum and 57-day milk. Moreover, the OP-1 purified from mammary gland extract is morphogenically-active and is also detected in the bloodstream. Maternal administration, via ingested milk, may be a natural delivery route of TGF-β superfamily proteins. Letterio, *et al*., *Science 264*: 1936-1938 (1994), report that TGF-β is present in murine milk, and that radiolabelled TGF-β is absorbed by gastrointestinal mucosa of suckling juveniles. Labeled, ingested TGF-β appears rapidly in intact form in the juveniles' body tissues, including lung, heart and liver. Finally, soluble form morphogen, *e.g.*, mature morphogen associated with the pro domain, is morphogenically-active. These findings, as well as those disclosed in the examples below, indicate that oral and parenteral administration are viable means for administering TGF-β superfamily proteins, including the morphogens, to an individual. In addition, while the mature forms of certain morphogens described herein typically are sparingly soluble, the morphogen form found in milk (and mammary gland extract and colostrum) is readily soluble, probably by association of the mature, morphogenically-active form with part or all of the pro domain of the expressed, full length polypeptide sequence and/or by association with one or more milk components. Accordingly, the compounds provided herein may also be associated with molecules capable of enhancing their solubility *in vitro* or *in vivo.*

Where the morphogen is intended for use as a therapeutic for disorders of the CNS, an additional problem must be addressed: overcoming the blood-brain barrier, the brain capillary wall structure that effectively screens out all but selected categories of substances present in the blood, preventing their passage into the brain. The blood-brain barrier can be bypassed effectively by direct infusion of the morphogen or morphogen-stimulating agent into the brain, or by intranasal administration or inhalation of formulations suitable for uptake and retrograde transport by olfactory neurons. Alternatively, the morphogen or morphogen-stimulating agent can be modified to enhance its transport across the blood-brain barrier. For example, truncated forms of the morphogen or a morphogen-stimulating agent may be most successful. Alternatively, the morphogens, inducers or agonists provided herein can be derivatized or conjugated to a lipophilic moiety or to a substance that is actively transported across the blood-brain barrier, using standard means known to those skilled in the art. See, for example, Pardridge, *Endocrine Reviews 7*: 314-330(1986) and U.S. Pat. No. 4,801,575.

The compounds provided herein may also be associated with molecules capable of targeting the morphogen, inducer or agonist to the desired tissue. For example, an antibody, antibody fragment, or other binding protein that interacts specifically with a surface molecule on cells of the desired tissue, may be used. Useful targeting molecules may be designed, for example, using the single chain binding site technology disclosed in U.S. Pat. No. 5,091,513. Targeting molecules can be covalently or non-covalently associated with the morphogen, inducer or agonist.

As will be appreciated by one of ordinary skill in the art, the formulated compositions contain therapeutically-effective amounts of the morphogen, morphogen inducers or agonists of morphogen receptors. That is, they contain an amount which provides appropriate concentrations of the agent to the affected nervous system tissue for a time sufficient to stimulate a detectable restoration of impaired central or peripheral nervous system function, up to and including a complete restoration thereof. As will be appreciated by those skilled in the art, these concentrations will vary depending upon a number of factors, including the biological efficacy of the selected agent, the chemical characteristics (*e.g.*, hydrophobicity) of the specific agent, the formulation thereof, including a mixture with one or more excipients, the administration route, and the treatment envisioned, including whether the active ingredient will be administered directly into a tissue site, or whether it will be administered systemically. The preferred dosage to be administered is also likely to depend on variables such as the condition of the diseased or damaged tissues, and the overall health status of the particular mammal. As a general matter, single, daily, biweekly or weekly dosages of 0.00001-1000 mg of a morphogen are sufficient, with 0.0001-100 mg being preferable, and 0.001 to 10 mg being even more preferable. Alternatively, a single, daily, biweekly or weekly dosage of 0.01-1000 µg/kg body weight, more preferably 0.01-10 mg/kg body weight, may be advantageously employed. The present effective dose can be administered in a single dose or in a plurality (two or more) of installment doses, as desired or considered appropriate under the specific circumstances. A bolus injection or diffusable infusion formulation can be used. If desired to facilitate repeated or frequent infusions, implantation of a semi-permanent stent (*e.g.*, intravenous, intraperitoneal, intracisternal or intracapsular) may be advisable.

The morphogens, inducers or agonists of the invention may, of course, be administered alone or in combination with other molecules known to be beneficial in the treatment of the conditions described herein. For example, various well-known growth factors, hormones, enzymes, therapeutic compositions, antibiotics, or other bioactive agents can also be administered with the morphogen. Thus, various known growth factors such as NGF, EGF, PDGF, IGF, FGF, TGF-α, and TGF-β, as well as enzymes, enzyme inhibitors, antioxidants, anti-inflammatory agents, free radical scavenging agents, antibiotics and/or chemoattractant/chemotactic factors, can be included in the present morphogen formulation.

### Example 1: Preparation of Soluble Morphogen Protein Solutions for In vivo Administration

### A. Aqueous Solutions

While the mature dimeric morphogenic proteins defined herein are typically sparingly soluble in physiological buffers, they can be solubilized to form injectable suspensions or solutions. One exemplary aqueous formulation containing a morphogen is made, for example, by dispersing the morphogen in 50% ethanol containing acetonitrile in 0.1% trifluoroacetic acid (TFA) or 0.1% HCl, or in an equivalent solvent. One volume of the resultant solution then is added, for example, to ten volumes of phosphate buffered saline (PBS), which further may include 0.1-0.2% human serum albumin (HSA) or a similar carrier protein. The resultant solution is preferably vortexed extensively to produce a physiologically acceptable morphogen formulation.

In another embodiment, the morphogen, including OP-1, is solubilized by reducing the pH of the solution. In one preferred formulation, the protein is solubilized in 0.2 mM acetate buffer, pH 4.5. containing 5% mannitol, to render the solution more isotonic. Other standard means for creating physiologically acceptable formulations are within the skill of the art.

### B. Soluble Complex Formulations

Another preferred form is a dimeric morphogenic protein comprising at least the C-terminal seven cysteine skeleton characteristic of the morphogen family, complexed with a peptide comprising a pro region of a member of the morphogen family, or a solubility-enhancing fragment thereof, or an allelic, phylogenetic or other sequence variant thereof. The solubility-enhancing fragment is any N-terminal or C-terminal fragment of the pro domain polypeptide of a member of the morphogen family that complexes with the mature polypeptide dimer to enhance the stability of the resulting soluble complex. Preferably, the dimeric morphogenic protein is complexed with two such pro domain peptides.

As described above and in published application WO 94/03600, the soluble complex form is isolated from the cell culture media (or a body fluid) under appropriate conditions. Alternatively, the complex is formulated *in vitro*.

Soluble morphogen complexes are isolated from conditioned media using a simple, three step chromatographic protocol performed in the absence of denaturants. The protocol involves running the media (or body fluid) over an affinity column, followed by ion exchange and gel filtration chromatographies generally as described in WO 94/03600. The affinity column described below is a Zn-IMAC column. The present example uses human OP-1, and is not intended to be limiting. The present protocol has general applicability to the purification of a variety of morphogens, all of which are anticipated to be isolatable using only minor modifications of the protocol described below. An alternative protocol also envisioned to have utility includes an immunoaffinity column, created using standard procedures and, for example, using antibody specific for a given morphogen pro domain (complexed, for example, to a protein A-conjugated Sepharose column). Protocols for developing immunoaffinity columns are well described in the art (see, for example, GUIDE TO PROTEIN PURIFICATION, M. Deutscher, ed., Academic Press, San Diego, 1990, particularly sections VII and XI thereof).

In this example, OP-1 was expressed in mammalian (CHO, Chinese hamster ovary) cells as described in the art (see, for example, international application US90/05903 (WO 91/05802). The CHO cell conditioned media containing 0.5% FBS is initially purified using Immobilized Metal-Ion Affinity Chromatography (IMAC). The soluble OP-1 complex from conditioned media binds very selectively to the Zn-IMAC resin and a high concentration of imidazole (50 mM imidazole, pH 8.0) is required for the effective elution of the bound complex. The Zn-IMAC purified soluble OP-1 is next applied to an S-Sepharose action-exchange column equilibrated in 20 mM NaPO₄ (pH 7.0) with 50 mM NaCl. The protein then is applied to a Sephacryl S-200HR column equilibrated in TBS. Using substantially the same protocol, soluble morphogens can also be isolated from one or more body fluids, including milk, serum, cerebrospinal fluid or peritoneal fluid.

The soluble OP-1 complex elutes with an apparent molecular weight of 110 kDa. This agrees well with the predicted composition of the soluble OP-1 complex, with one mature OP-1 dimer (35-36 kDa) associated with two pro domains (39 kDa each). Purity of the final complex can be verified by running the appropriate fraction in a reduced 15% polyacrylamide gel.

As an alternative to purifying soluble complexes from culture media or a body fluid, soluble complexes can be formulated from purified pro domains and mature dimeric species. Successful complex formation apparently requires association of the components under denaturing conditions sufficient to relax the folded structure of these molecules, without affecting disulfide bonds. Preferably, the denaturing conditions mimic the environment of an intracellular vesicle sufficiently such that the cleaved pro domain polypeptide has an opportunity to associate with the mature dimeric protein under relaxed folding conditions. The concentration of denaturant in the solution then is decreased in a controlled, preferably step-wise manner, so as to allow proper refolding of the dimer and pro domain peptides, while maintaining the association of the pro domain peptides with the mature dimer. Useful denaturants include 4-6 M urea or guanidine hydrochloride (GuHCl), in buffered solutions of pH 4-10, preferably pH 6-8. The soluble complex then is formed by controlled dialysis or dilution into a solution having a final denaturant concentration of less than 0.1-2M urea or GuHCl, preferably 1-2 M urea or GuHCl, which then preferably can be diluted into a physiological buffer. Protein purification/renaturing procedures and considerations are well described in the art, and details for developing a suitable renaturing protocol readily can be determined by one having ordinary skill in the art. One useful text on the subject is GUIDE TO PROTEIN PURIFICATION, M. Deutscher, ed., Academic Press, San Diego, 1990, particularly section V. Complex formation may also be aided by addition of one or more chaperone proteins.

The stability of the highly purified soluble morphogen complex in a physiological buffer, *e.g.*, Tris-buffered saline (TBS) and phosphate-buffered saline (PBS), can be enhanced by any of a number of means, including any one or more of three classes of additives. These additives include basic amino acids (*e.g.*, L-arginine, lysine and betaine); nonionic detergents (*e.g.*, Tween 80 or NonIdet P-120); and carrier proteins (*e.g.*, serum albumin and casein). Useful concentrations of these additives include 1-100 mM, preferably 10-70 mM, including 50 mM, basic amino acid; 0.01-1.0%, preferably 0.05-0.2%, including 0.1% (v/v) nonionic detergent; and 0.01-1.0%, preferably 0.05-0.2%, including 0.1% (w/v) carrier protein.

### Example 2. Identification of Morphogen-Expressing Tissue

Determination of the tissue distribution of morphogens may be used to identify different morphogens expressed in a given tissue, as well as to identify new, related morphogens. Tissue distribution also may be used to identify useful morphogen-producing tissue for use in screening and identifying candidate morphogen-inducing agents. The morphogens (or their mRNA transcripts) readily are identified in different tissues using standard methodologies and minor modifications thereof in tissues where expression may be low. For example, protein distribution may be determined using standard Western blot analysis or immunofluorescent techniques, and antibodies specific to the morphogen or morphogens of interest. Similarly, the distribution of morphogen transcripts may be determined using standard Northern hybridization protocols and transcript-specific probes.

Any probe capable of hybridizing specifically to a transcript, and distinguishing the transcript of interest from other, related transcripts may be used. Because the morphogens described herein share such high sequence homology in their active, C-terminal domains, the tissue distribution of a specific morphogen transcript may best be determined using a probe specific for the pro region of the immature protein and/or the N-terminal region of the mature protein. Another useful sequence is the 3' non-coding region flanking and immediately following the stop codon. These portions of the sequence vary substantially among the morphogens useful in this invention, and accordingly, are deemed specific for each protein. For example, a particularly useful Vgr-1-specific probe sequence is the PvuII-SacI fragment, a 265 bp fragment encoding both a portion of the untranslated pro region and the N-terminus of the mature sequence (see Lyons, *et al.*, (1989) *PNAS* 86: 4554-4558 for a description of the cDNA sequence). Similarly, particularly useful mOP-1-specific probe sequences are the BstXI-BglI fragment, a 0.68 Kb sequence that covers approximately two-thirds of the mOP-1 pro region; a StuI-StuI fragment, a 0.2 Kb sequence immediately upstream of the 7-cysteine domain; and the Earl-Pstl fragment, a 0.3 Kb fragment containing a portion of the 3'-untranslated sequence. Similar approaches may be used, for example, with hOP-1 or human or mouse OP-2.

Using these morphogen-specific probes, which may be synthetically engineered or obtained from cloned sequences, morphogen transcripts can be identified in mammalian tissue, using standard methodologies well known to those having ordinary skill in the art. Briefly, total RNA is prepared from various adult murine tissues (*e.g.*, liver, kidney, testis, heart, brain, thymus and stomach) by a standard methodology such as by the method of Chomczyaski, *et al.,* ((1987) *Anal. Biochem* 162: 156-159) and described below. Poly (A)+ RNA is prepared by using oligo (dT)-cellulose chromatography (*e.g*., Type 7, from Pharmacia LKB Biotechnology, Inc.). Poly (A)+ RNA (generally 15 mg) from each tissue is fractionated on a 1% agarose/formaldehyde gel and transferred onto a Nytran membrane (Schleicher & Schuell). Following the transfer, the membrane is baked at 80°C and the RNA is cross-linked under UV light (generally 30 seconds at 1 mW/cm²). Prior to hybridization, the appropriate probe is denatured by heating. The hybridization is carried out in a lucite cylinder rotating in a roller bottle apparatus at approximately 1 rev/min for approximately 15 hours at 37°C using a hybridization mix of 40% formamide, 5 x Denhardts, 5 x SSPE, and 0.1% SDS. Following hybridization, the non-specific counts are washed off the filters in 0.1 x SSPE, 0.1% SDS at 50°C.

Examples demonstrating the tissue distribution of various morphogens, including Vgr-1, OP-1, BMP2, BMP3, BMP4, BMP5, GDF-1, and OP-2 in developing and adult tissue are disclosed in co-pending USSN 752,764, and in Ozkaynak. *et al.*, (1991) *Biochem. Biophys. Res. Commn. 179*: 116-123, and Ozkaynak, *et al*., (1992) 267 *J. Biol. Chem.* 25220-25227, the disclosures of which are incorporated herein by reference. Using the general probing methodology described herein, Northern blot hybridizations using probes specific for these morphogens to probe brain, spleen, lung, heart, liver and kidney tissue indicate that kidney-related tissue appears to be the primary expression source for OP-1, with brain, heart and lung tissues being secondary sources. Lung tissue appears to be the primary tissue expression source for Vgr-1, BMP5, BMP4 and BMP3. Lower levels of Vgr-1 also are seen in kidney and heart tissue, while the liver appears to be a secondary expression source for BMP5, and the spleen appears to be a secondary expression source for BMP4. GDF-1 appears to be expressed primarily in brain tissue. To date, OP-2 appears to be expressed primarily in early embryonic tissue. Specifically, Northern blots of 8-day murine embryos show abundant OP2 expression. Expression is reduced significantly in 17-day embryos and is not detected in post-natal animals.

### Example 3. Characterization of Morphogen Expression in Embryonic Tissues

The expression pattern of morphogen in embryonic tissue has been examined using rat and human embryonic tissues.

Immunohistochemical staining of polyclonal and monoclonal antibodies against recombinant, mature human OP-1 was used to examine the distribution of OP-1 in serial sections of human embryos ranging from 5 to 14 weeks of gestation. Vukicevic, *et al.*, (1994), 198 *Biochem. Biophys. Res. Comm.* 693-700. The authors observed that OP-1 is associated with tissues derived from embryonic mesoderm and neuroectoderm, and that particularly high accumulations are found during organogenesis at epithelial-mesenchymal boundaries. OP-1 was found in tissues of the limbs during digit formation, in the dermis, at sites of endochondral and intramembranous bone formation, in neurons of the telencephalon from which the cerebral hemispheres develop, and in many other sites of particular morphogenetic activity.

Additional studies by these authors, unpublished, demonstrate that the meninges and the cells of the subpial granular layer of the telencephalon stained strongly for OP-1 protein as early as 6 weeks of gestation. Intensive staining was also detected in neuronal cells of the telencephalic cortical plate. No specific observation was, however, reported of OP-1 protein in the vicinity of developing human eye tissues.

Other workers have examined the expression pattern of OP-1 in human and mouse development by Northern hybridization and *in situ* hybridization. (Helder, *et al*., (1995) 43 *J. Histochemistry and Cytochemistry* 1035-1044). Northern hybridization of 12-14 week human embryo tissues using an OP-1 transcript specific probe, demonstrated expression of OP-1 in a number of embryonic tissues, including, kidney, brain, heart, and lung. OP-1 transcripts were detected at 6 weeks gestation in the metanephric mesenchyme surrounding the epithelium of the ureteric bud. At later developmental states, high levels of OP-1 mRNA were present in the developing glomeruli and less in adjacent convoluted tubules and straight collecting ducts. In 7 week gestation human embryos, OP-1 transcripts were localized to the perichondrium of the cartilaginous long bones. At later developmental stages, OP-1 transcripts were found in the periosteum/osteoblast layer of the osteogenic zone. OP-1 transcripts also are present in cytotrophoblasts of human embryos at 3 to 12 gestational weeks in muscle, and in gastrointestinal mucosa between 6 and 14 weeks.

The distribution of OP-1 transcripts in 9.5 and 10.5 day post coital (p.c.) mouse embryos was examined by *in situ* hybridzation of serial sections. At 9.5 days p.c., a strong OP-1 transcript signal was detected in the ectodermal epithelium of the developing limb buds. At 10.5 days p.c., OP-1 transcripts were localized in the ectodermal epithelium of the fore and hindlimbs, particularly in the apical ectodermal ridge, olfactory plaque, ophthalmic neural crest, diencephalon, neural tube and heart. Less intense, uniformly distributed expression was also detected in the underlying mesenchyme of the forelimb buds, and uniform expression was observed in the limb bud mesoderm. In older embryos, 12.5-18.5 days p.c., several craniofacial structures showed expression of OP-1 transcripts, predominantly skin, hair follicles, nasal epithelium, teeth, calvaria, meninges, choroid plexus, and salivary glands. In 12.5-14.5 days p.c. mouse embryos, OP-1 transcripts were observed around the cartilage precondensations of developing bones.

The Helder study also reports that *in situ* hybridization has revealed BMP-2 expression in the apical ectodermal ridge and the pre-cartilaginous mesenchyme on the early limb bud, and high levels of BMP-6 expression in hypertrophic chondrocytes of more developed bone structures. These observations are understood herein to be consistent with the view that morphogen-responsiveness of developing neuroectodermal tissues is not unique to OP-1.

Immunohistochemistry and *in situ* hybridization techniques were employed to visualize the distribution of endogenous OP-1 mRNA and protein in head sections prepared from day 11.5, 12.5 and 13.5 rat embryos. (Solursh, *et al.*, (1996), 218 *Biochem. Biophys. Res. Comm.* 438-443). These techniques demonstrated that, on day 11.5, OP-1 message was widely expressed in neural epithelium and was prominent in the optic vesicle. Some immunohistochemical staining was observed within the optic vesicle, however, significant amounts of OP-1 protein were not detected until contact of the optic vesicle with the presumptive lens placode ectoderm at day 12.5, at which time the ectoderm displayed positive staining around the optic vesicle. By day 12.5, expression of the OP-1 message was restricted to the presumptive neural retina of the optic cup and invaginating placode, and immunoreactivity of the placode grew more intense as it invaginated. OP-1 expression persisted in the developing lens epithelium through day 13.5, by which time the lens had separated from the presumptive corneal epithelium. At this stage, the expression of OP-1 message became also evident in the cornea.

Morphogens also have been identified in developing rat eye tissue, as determined by exposure of whole rat embryos to OP-1 blocking antibody. (Solursh, *et al.*, *ibid.*). Whole rat embryos were cultured over a 72 hour period from the primitive streak stages to early limb states. The rat embryos were exposed to either 10 mg/ml of a non-reactive IgG or 10mg/ml of blocking antibody specifically reactive with OP-1. The control embryos developed completely normally, while those exposed to OP-1 blocking antibody consistently exhibited a number of abnormalities. Specifically, rat embryos which were exposed to OP-1 blocking antibody were reduced in size overall, had reduced facial size and smaller hearts. The majority of these embryos also lacked eyes. Inspection of histochemically stained sections through the head region of embryos exposed to the OP-1 blocking antibody revealed that the neural retina had scarcely developed.

Studies using homozygous mutant mice, lacking a functional gene encoding OP-1, extended the foregoing eye development studies. (Dudley, *et al*., (1995), 9 *Genes & Dev.* 2795-2807; Luo, *et al.*, (1995), 9 *Genes & Dev.* 2808-2820). Dudley reports the creation of OP-1 nullizygous mice (so-called "knockout" mice) by embryonic stem cell technology. Dudley reports that the knockout mice exhibit renal dysplasia and a range of eye defects, including variable degrees of micropthamia and anophthalmia. Luo similarly reports an assessment of morphological abnormalities in OP- 1-nullizygous mice. Luo emphasizes that the reported ophthalmic abnormalities exhibit variable penetrance. Indeed, some mice were found to be ophthalmologically normal. Dudley surmises that OP-1 "acts as a localized growth factor regulating the survival or maintenance of differentiated cell types essential for the maturation of both" eye and kidney. The initial induction events responsible for eye morphogenesis were intact, however the eye primordia degenerated bilaterally in 60% of knockout embryos. An additional 40% displayed bilateral microphthalmia or unilateral microphthalmia in combination with unilateral anopthalmia, At embryonic day 9.5, induction of the lens placode and invagination of the lens vesicle were observed in mutant embryos. However, by day 14,5 the majority of the mutant embryos exhibited profound bilateral deterioration of the developing eye structures. At this stage the only recognizable eye tissue was described as a small mass of disorganized pigmented retinal epithelium associated with the remnant of the optic nerve.

These studies indicate the possibility of both age-dependent and tissue-dependent differences in morphogen expression. Further, these studies implicate morphogens to be involved in pattern formation during natural morphogenesis of the developing embryo.

### Example 4. Use of Morphogen For Treatment of Corneal Wounds

The ability of the morphogens described herein to treat comeal wounds can be measured according to the following procedure, described by Leibowitz, *et al*., (1990), 108 *Arch. Ophthalmol*. 734-737. Skilled artisans will understand and appreciate that the specific procedure of Liebowitz. *et al*., can be routinely modified and adapted to better serve the artisan's illustrative purpose. Enhancement in wound healing in the cornea has significant therapeutic application for all types of corneal surgery, as discussed previously herein.

New Zealand albino rabbits are anesthetized intramuscularly, and, concurrently, the surface of one eye, chosen randomly, is anaesthetized, for example by instillation of 0.5% proparacaine hydrochloride. Under direct visualization through an operating microscope, a 9-mm groove, approximately 0.5 corneal thickness in depth, should be made across the center of the cornea with a surgical blade. A strabismus caliper is used to scratch shallow parallel secondary grooves 2 mm on either side of initial primary groove. The strabismus caliper then is used to mark the primary groove into four equal parts along its length to designate the location of the closing sutures. The surgical blade, edge upward, is introduced through one end of the primary groove into the anterior chamber, carried across the anterior chamber, and caused to emerge at the opposite end of the groove, creating a perforating incision. Four 10-0 nylon-interrupted sutures, placed at the marks along the incision, are used to close the wound. In suturing, the needle can be introduced into the superior secondary groove, passed through full-thickness cornea into the anterior chamber, caused to emerge through the inferior secondary groove, and tied on the corneal surface at the inferior corneal groove. On completion of the surgical procedure, 0.5% erythromycin ophthalmic ointment can be placed over the wound.

Topical therapy is started immediately after surgery. Control animals are treated with carrier while the experimental animals are treated with morphogen in carrier. Appropriate carriers are discussed above. Morphogen is administered once, twice or four times a day for a period, for example, 6 to 9 days. Topical therapy is preferred, as it has been observed to offer the single most effective route for suppression of corneal inflammation. Frangie, *et al*., (1993), 33 *Int. Opthalmol. Clin*. 9-29. The capacity of the conjunctival cul-de-sac is limited, thus increasing the volume of a topical dose beyond 50 µl does not enhance drug delivery. Amounts in excess of 50 µl pass through the puncta into the nasolacrimal system or find their way over the eyelid margin and down the cheek. One may increase the total drug delivery either by increasing the frequency of topical application or by increasing the concentration of the drug.

If desired, prophylactic therapy also can be assessed using the Liebowitz corneal wounding model. To test prophylactic efficacy, morphogen is administered systemically, *e.g*., 2 to 24 hours prior to initiating surgery.

On completion of the treatment protocol, all rabbits are killed with an overdose of intravenously administered pentobarbital sodium. Under visualization through an operating microscope, a 25-gauge disposable needle is inserted through perilimbal clear cornea at the 6-o'clock meridian into the anterior chamber. The needle is connected by means of plastic tubing to a pressure transducer and a syringe-drive pump. The sutures are cut with an micro-surgical blade and removed with tying forceps. Buffered saline is pumped into the anterior chamber at a rate of 0.35 mL/min, and the output of the pressure transducer is recorded on a polygraph. The increase in intraocular pressure, and the point at which the pressure falls, is recorded by the polygraph. The abrupt fall in pressure indicates rupture of the corneal wound and is recorded as "wound strength". Morphogen treatment or prophylaxis of the eye is expected to increase the wound strength of the corneal wounds, relative to wound strength in untreated eyes.

### Example 5. Use of Morphogen to Treat Abnormal Neovascularization

Abnormal neovascularization in the eye is associated with a number of disease conditions. Schultz, *et al*., (1991) 5 *Eye* 170-180. Early *in vitro* experiments demonstrated that aliquots of vitrectomy fluid from human eyes with neovascularization stimulated blood vessel growth on chick chorioallantonic membrane, and stimulated endothelial cell division. Both stimulatory and inhibitory angiogenic activities have been detected in vitreous samples, suggesting that an imbalance of these activities contributes to neovascularization.

The morphogens of the present invention can be used in treating ophthalmic and other loci of neovascularization, including neovascularization associated with, for example: cancer, solid tumors, arthritis, diabetes, arteriosclerosis, angiofibroma, arteriovenous malformations, corneal graft neovascularization, delayed wound healing, diabetic retinopathy, age related macular degeneration, granulations, burns, hemangioma, hemophilic joints, hypertrophic scars, neovascular glaucoma, nonunion fractures, Osler-Weber Syndrome, psoriasis, pyogenic granuloma, retrolental fibroplasia, pterigium, scleroderma, trachoma, vascular adhesions, idiopathic ocular neovascularization, parasitic diseases, hypertrophy following surgery, inhibition of hair growth, inhibition of ovulation and corpus luteum formation and inhibition of embryo implantation and development in the uterus.

Morphogens are particularly useful in preventing and/or treating ophthalmic neovascularization, including but not limited to: retinal disease associated neovascularization (diabetic retinopathy, chronic glaucoma, retinal detachment, sickle cell retinopathy, age related macular degeneration); rubeosis iritis: inflammatory diseases; chronic uveitis; neoplasms (retinoblastoma, pseudoglima); Fuchs' heterochromic iridocyclitis; neovascular glaucoma; corneal neovascularization (inflammatory, transplantation; related developmental hypoplasia of the iris); neovascularization resulting following combined vitrectomy and lensectomy; vascular diseases (retinal ischemia, choroidal vascular insufficiency, choroidal thrombosis, carotid artery ischemia): pterigium; neovascularation of the optic nerve; and neovascularization due to traumatic penetration of the eye or contusive ocular injury.

The ability of morphogen to treat diseases involving neovascularization can be assessed, for example, using a murine model of proliferative retinopathy as described by Robinson, *et al.*, (1996), 93 *Proc. Natl. Acad. Sci. USA*, 4851-4856. In this model, postnatal day 7 mice are exposed for 5 days to hyperoxic conditions (75 ± 2% oxygen) in a sealed incubator, according to known methods. After 5 days, the twelve day old mice are returned to room air. Return of animals to room air is believed to result in relative hypoxia of the non-perfused areas of the retina. Retinal neovascularization then occurs in 100% of the animals. Maximal retinal neovascularization is observed 5 days after return to room air.

To assess protective effects, morphogen is injected into anesthetized vitreous humor, to deliver 0.5 µl of morphogen solution diluted in suitable carrier. Repeat injections are done through a previously unmanipulated section of the limbus. Injections are performed immediately upon removal of oxygen, on twelve day old mice, and are repeated two days later, on 14 day old mice. Animals are sacrificed on day 17 by injection with a lethal dose of anesthesia followed by cardiac perfusion with 4% paraformalhyde (Sigma) in PBS. If desired, this dosing schedule can be modified to allow assessment of acute or chronic treatment following the establishment of neovasculature.

Following sacrifice, the eyes are enucleated, fixed in 4% paraformaldehyde, and embedded in paraffin. Serial 6-µm sections of the whole eyes are cut sagittally parallel to the optic nerve, and stained with hematoxylin and periodic acid/Schiff stain according to a standardized protocol. The extent of neovascularization in the treated and control eyes is determined by counting neovascular cell nuclei extending through the internal limiting membrane into the vitreous. All counting should be done using a masked protocol. For each eye, 10 intact sections of equal length, each 30 µm apart, should be evaluated. The mean number of neovasacular nuclei per section per eye is then determined. The extent of neovascularization resulting in morphogen-treated eyes is then compared with that found in control eyes using Student's *t* test or the Wilcox Signed Rank test. Eyes found to have intraocular inflammation, infection, or retinal detachment should be excluded from analysis.

Angiostatic activity of the test compounds (*e.g*., morphogens, morphogen inducers or morphogen agonists) also can be evaluated in the chicken embryo chorioallantoic membrane model of neovascularization as described by McNatt, *et al*., (1992) 42 *J. Steroid Biochem. Mol. Biol*. 687-693. Briefly, morphogen is added to a 10 µL bead containing 1% agarose and liposomes (2.5% dimyristoyl phosphatidyl choline) and placed on the chorioallantoic membrane of a shell-less, 5-6 day chick embryo. After an additional 2 days of incubation at 37°C, the zone of avascularity surrounding the dose bead is evaluated. Angiostatic activity is expressed as the angiostatic response frequency per nmole of test compound.

Morphogen is expected to suppress, or induce regression of neovascularization in either of the foregoing model systems.

### Example 6. Morphogen Treatment of Retinal Disorders

Morphogen also can be used to treat retinal disorders, particularly for treatment of macular degeneration and holes, where it is anticipated to promote healing and significantly improve vision. The treatment also may be used on retinal holes, tears, and detachment. These disorders are characterized by loss of visual acuity.

Morphogen may be administered to such patients by any of a variety of routes and formulations known in the art, as discussed above. Slow-release of morphogen may be particularly beneficial for the treatment of retinal detachment and macular holes. Hence, morphogen may be administered in a slow-release device embedded in the tissue stroma or in a compartment adjacent to the affected tissue. For example, an effective concentration of morphogen in a pellet of ethylene vinyl copolymer 2 mm in diameter could be surgically implanted in the vitreous cavity or suprachoroidal space to release morphogen over time.

For example, morphogen can be used for treatment of complicated retinal detachment. Retinal detachment is a complication known to result from proliferative diabetic retinopathy and proliferative vitreoretinopathy. Currently such retinal detachment is treated by administration of silicone oil, which treatment can cause complications including corneal decompensation, lens opacification, intraocular pressure elevation and hypotony. Treatment with silicone oil is described in Yeo, *et al.*, (1987) 94 *Ophthalmology* 1109-1113. Treatment of detached retinas with morphogen is anticipated to obviate the limitations of known treatments.

Eyes to be treated with morphogen undergo complete pre- and post-operative ocular examination including visual acuity testing, intraocular pressure measurements, slit-lamp bio-microscopy, and binocular indirect ophthalmoscopy. Surgical strategy will vary dependent upon the exact vitreoretinal anatomy, as is known in the art. Generally, all vitrectomies are performed with standard three-port instrumentation. Vitrectomy optionally is performed prior to morphogen injection. Revision of the scleral buckle, periretinal membrane dissection, and relaxing retinotomies are used to relieve tractional forces. Fluid-air exchange and removal of subretinal fluid are then performed to flatten the retina. Morphogen is injected through the vitrectomy infusion cannula, and the air simultaneously displaced out of the eye via a 30-gauge needle introduced through the pars plana. Morphogen preferably is formulated in a viscous carrier and the bubble of morphogen is brought up to the posterior surface of the lens in phakic and pseudophakic eyes, and in aphakic eyes (eyes from which the lens has been removed) the morphogen is brought up to just behind the pupilary plane.

Administration of morphogen is anticipated to promote reattachment of the retina and regeneration of lost retinal tissues. In addition, administration of morphogen should inhibit reproliferation of fibrovascular tissue, and inhibit neovascularization.

Morphogen also can be used to treat macular holes by the placement of a concentrated solution of morphogen on the macular hole itself and/or the edges of the macular hole. Such treatment is expected to provide improvement of vision and healing by decreasing the thickness of the edge of the hole. The edges of the hole will appear to adhere to choroid or reconnect with the posterior hyaloid membrane. The morphogen can be applied using known surgical techniques, as described above.

### Example 7. Human Fetal Nervous System Progenitor Cells

Suitable sources of progenitor cells useful herein include cells or tissue derived from a human fetus. Central nervous system tissue can be obtained from the developing central nervous system of fetuses available from elective abortion. A suitable population of progenitor cells can be obtained from any specific region of the human fetal nervous system. Exemplary specific regions are ventral forebrain. cerebral cortex, cerebellum. midbrain. brainstem, neural tube or spinal cord, and neural crest.

Morphogen induction of cell differentiation of neural progenitor cells has been observed for at least OP-1 and BMP-4. Liem, *et al.*, (1995, 82 *Cell,* 969-979), demonstrates that OP-1 and BMP-4 are expressed in epidermal ectoderm and are mediators for epidermal ectoderm induction of dorsal cell differentiation in neural plate cells. More specifically, both OP-1 and BMP-4 were demonstrated to induce dorsal cell differentiation in neural plate cells. This indicates that OP-1 and/or BMP-4 is responsible for the production of neural crest cells and other primitive components of the developing vertebrate central and peripheral nervous systems.

As used herein, a nervous system "progenitor" cell is an uncommitted neural or neuroectodermal cell that is capable of producing daughter cells competent to undergo differentiation into, *e.g.*, neurons, glial cells photoreceptors, ciliary cells, chemoreceptors, and the like. As used herein, the phrase "central nervous system" means the brain and spinal cord or fetal tissue that gives rise to those tissues in the adult.

Methods for the preparation of progenitor cells are described in WO 96/04368. Central nervous system tissue is dissociated from the fetus using standard dissociation techniques well known in the art. By way of example, cells are physically dissociated from specific regions of the central nervous system by blunt dissection. Alternatively, cells are obtained by chemical means including treatment of tissue with enzymes such as trypsin, collagenase and the like. Central nervous system cells, once dissociated from tissue, are typically purified using standard procedures well known in the art. Similar techniques can be used to obtain disassociated preparations of peripheral nervous system cells and/or neural crest cells.

Human fetal nervous system cells, cultured as described below, form cellular masses that comprise progenitor cells. Those cellular masses are typically spherical in nature. The progenitor cells in those masses can be dissociated and further propagated (*e.g.*, subcultured) to form further masses of proliferating cells. Using this method human fetal progenitor cells can be propagated for extended periods of time. Exposure of the cultured cell masses specifically described in WO96/04368 to morphogen is anticipated to enrich the masses in progenitor cells.

By way of specific example, progenitor cells can be obtained and cultured as follows, as described in WO 96/04368. Human fetal brain tissue is obtained from routine therapeutic abortions. Evacuated tissue fragments from 6-8 week post-conception fetuses are collected directly into sterile ice-cold isotonic saline with heparin (10µg/ml). Ventral forebrain is dissected under stereomicroscopic observation in a laminar flow containment hood, and transferred to a solution of 0.05% (w/v) trypsin (Type XIII, Sigma) in calcium- and magnesium-free Hank's Balanced Salt Solution (CMF-HBSS) for 20 minutes at 37°C.

This incubation is followed by 4 washes with 0.01% deoxyribonuclease (DNase I, sigma) in complete HBSS. The tissue is then dissociated to a suspension of single cells in 0.01% DNase I in CMF-HBSS by repeated gentle passage through fire-polished Pasteur pipettes of decreasing bore size. Viability of the dissociated cells is assessed by exclusion of ethidium bromide, after incubation in a solution of acridine orange and ethidium bromide (1µg/ml each in HBSS). Cells are counted in a hemocytometer using standard fluorescein and rhodamine fluorescence filters to detect acridine orange- and ethidium bromide-containing cells respectively.

Cells are plated at 2.0 x 10³ viable cells per mm² in 24-well uncoated Nunc tissue culture dishes. Progenitor cell culture medium can be composed of a 3: 1 mixture of Dulbecco's Minimum Essential Medium (DMEM: Gibco) and Ham's F-12 (Gibco), with 5% (v/v) horse serum (Gibco), epidermal growth factor (Upstate Biochemicals, 20 ng/ml), insulin (Sigma, 10µg/ml), transferrin (Sigma, 200 µg/ml, progesterone (Sigma, 40 mM), putrescine (Sigma, 200 µM) and solium selenite (Sigma, 60 nM). This medium can be further supplemented with IGF-I (Upstate Biochemicals, 100 ng/ml). Cells are fed at 3-4 day intervals. At intervals of 30-60 days in vitro (DIV), resulting spherical call masses are dissociated as above, and cells related at a density of 2 x 10³ viable cells per mm². Human fetal ventral forebrain tissue plated in medium containing EGF at a density of 2 x 10³ cells per mm² on uncoated tissue culture plastic form spherical cellular masses 0.2-1.5 mm in diameter after 30-60 DIV. The formation of these masses is greatly enhanced in medium containing EGF and IGF.

After dissociating and relating the cells of these masses, 'secondary' cell masses form; growth of secondary cells masses is less rapid than that of primary cell masses. After a further 30-60 cell divisions, secondary masses can be similarly dissociated to produce 'tertiary' masses of proliferating cells. Such passage can be repeated at least two times.

It is anticipated that progenitor-enriched cell populations produced as described above will be useful in the production, according to the present invention, of replacement sense organ tissue, especially ocular tissue, when treated with a morphogen, inducer or agonist and implanted in a mammal in need of functional replacement tissue. Optionally, such cells can be used to produce functional sensory tissue in a recipient host mammal unable by reason of a congenital, hereditary or acquired defect, to produce functional regenerated tissue when simply treated with a morphogen. Thus, for example, genomically wild-type progenitor cells can be used to produce functional retinal tissue in a mammal afflicted with retinitis pigmentosa. choroiderema or a similar degenerative condition.

### Example 8. Morphogen Treatment of Ophthalmic Inflammation

Morphogens are useful in the treatment of inflammation as described in WO 93/04692. Morphogen also can be used specifically to treat external ocular inflammation, *e.g.*, conjunctivitis, episcleritis, scleritis, or uveitis (inflammation of the iris, ciliary body, or choroid) by instillation of a solution of morphogen in an eye. External ocular inflammation can be due to the presence of a bacterium, fungus, virus or parasite in the ocular tissue. Uveitis can be due to systemic disease or the presence of a bacterium, fungus, virus or parasite in the uveal tissues. Pre- and post-treatment clinical evaluation of subjects will provide an assessment of symptoms, *e.g.*, discomfort, acute ocular pain, tearing, photophobia, visual acuity change, and itching, and of signs, *e.g.,* erythema, discharge, palpebral conjunctival inflammation, bulbar conjunctival inflammation, exudation, limbal inflammation, corneal epithelial disease, and focal stromal infiltrates. Following treatment with morphogen the symptoms and signs of ophthalmic inflammation should improve, and upon completion of morphogen treatment, substantially normal function should be restored to the eye. Further, the subjects should experience no substantial side effects or adverse experiences from instillation of morphogen.

The conjunctival sac is able to retain approximately 50 µl of fluid, therefore morphogen is formulated appropriately such that an effective concentration of morphogen will be present in each 50 µl of fluid. The frequency and duration of treatment can be readily ascertained by those of ordinary skill in the art. For example, for treatment a two drop dose of morphogen solution can be used even though this volume is greater than the volume that the conjunctival sac is able to retain because such volume will insure that each treated eye will receive a full dose of morphogen.

### Example 9. Morphogen Treatment of Corneal Inflammation

In many types of acute keratitis, large numbers of polymorphonuclear leukocytes (PMN's) invade the cornea. PMNs are known to play a central role in the inflammatory process through mechanisms, such as, release of lysosomal enzymes, arachidonic acid metabolites and oxygen-derived free radicals. Keratitis occurs as a result of many forms of ophthalmic treatment, *e.g.*, surgery or laser therapy, and retards the wound healing process. Administration of morphogen to ophthalmic tissue prior to or after onset of inflammation can prevent or suppress the invasion of PMNs, and concomitant keratitis, thereby enhancing regenerative wound healing.

The effectiveness of morphogen to treat keratitis in humans can be assessed using an established animal model. (Leibowitz, *et al.*, (1986) 27 *Invest. Ophthalmol. Vis. Sci.* 1226-1229,). Briefly, rabbits are administered three intravenous injections of 0.05 µCi/kg of tritiated thymidine at 24 hour intervals. Corneal inflammation is then induced by an intracomeal injection of 0.03 ml clove oil into an anesthetized animal. Morphogen is administered topically to each eye. The dosage and frequency of application can be readily determined by those of ordinary skill in the art. In some animals morphogen should be administered from a time commencing prior to administration of the clove oil, and in other animals morphogen should be administered from a time commencing after the administration of the clove oil. A comparison of such treatment results will allow determination of the effectiveness of morphogen to prevent the inflammatory response and to treat inflamed ophthalmic tissues. The effectiveness of morphogen treatment is quantitated by known methods. Briefly a 10 mm full-thickness corneal button is removed from each eye by trephination and the tissue sample is solubilized in a commercially available solubilizing agent. The solubilized sample is counted for tritium to quantitatively measure the amount of radioactivity in the cornea. (See, Leibowitz, *et al*., (1974) 92 *Arch. Ophthalmol*. 427; Leibowitz. *et al.*, (1974) 13 *Invest. Ophthalmol*. 757) The quantity of radioactivity measured in each sample is proportional to the effectiveness of morphogen treatment. That is, invasion of the corneal tissue of PMNs will reduce the quantity of radioactivity measured in that sample, thus high levels of remaining radioactivity when compared to samples derived from control animals indicates a suppression or prevention of invasion by PMNs.

### Example 10. Use of Morphogen For Sensory Hair Cell Regeneration

The inner ear can be divided into two sensory organs: the auditory organ for sound processing and vestibular organ for sensing orientation and motion. Degeneration of auditory and vestibular function in most cases is due to the loss of the sensory hair cells in these organs. Hair cells do not actually have hair but are sensory cells which display sterocilia and kinocilium on the apical surface of the sensory epithelia which are able to transduce mechanical auditory simulation from the outer and middle ear as well as gravity and motion into electrical signals. Attached to these cells are neurons which transmit these signals to the brain. Loss of sensory epithlium of the auditory organ leads to reduction in auditory function and loss of hearing. Loss of corresponding sensory structures in the vestibular sensory epithelium leads to disequilibrium and vertigo. Therefore, compositions capable of enhancing in sensory hair cell regeneration and preventing sensory hair cell degeneration have significant therapeutic application for all types of hearing loss and vestibular dysfunctions, as discussed previously herein.

The purpose of the present example is to assess the ability of morphogens to treat loss of sensory hair cells and the ability of morphogens to prevent loss of sensory hair cells. The effects of morphogens on sensory hair cells can be measured according to the procedures described below. Skilled artisans will understand and appreciate that these specific procedures can be routinely modified and adapted to better serve the artisan's illustrative purpose.

### 10.1 Effects of Morphogen on Sensory Hair Cells in Chick Cochlear Epithelium After Aminoglycoside Toxicity

The posthatch chick cochlear epithelium is an excellent model for studying hair cell regeneration. Damage to sensory hair cells in chick cochlear epithelium can be measured according to the procedures described by Stone, *et al*., *J. Neurosci. 16:* 6157-6174 (1996). White Leghorn chicks (Gallus domesticus) may be purchased from H & N International (Redmond, WA) and housed in heated brooders with ample food and water. Chicks between 5 and 7 days posthatch (40-55 gm) are given a single intraperitoneal injection of O gentamicin (400 mg/kg; Lyphomed, Deerfield, IL). Age-matched control chicks did not receive a gentamicin injection. Skilled artisans will understand and appreciate that sensory hair cells can also be damaged by exposure to other ototoxic drugs such as antitumor agents such as cisplatin [Kopke, *et al., Am. J. Otology 18*: 559-571 (1997)] or by ototoxic insult from resulting from exposure to frequency-specific otoacoustic emissions [Sie and Norton, *Otolaryngol. Head Neck Surg. 116*: 585-592 (1997)].

In one group of animals, the prophylactic effects of morphogens on sensory hair cells exposed to toxic aminoglycoside are assessed by administering OP-1 (0, 250, 1,000 or 2,000 µg/kg) to the chicks 10 min or one hour prior to the gentamicin injection. In another group of animals, the effects of morphogens on damaged sensory hair cells are assessed by administering OP-1 (0, 250, 1,000 or 2,000 µg/kg) to the chicks 1, 24, or 48 hours after the gentamicin injection.

At 2, 4, 7 or 10 days after the gentamicin injection, chicks are euthanized by an intraperitoncal injection of sodium pentobarbital and then are decapitated. Cochlear ducts are removed and placed in ice-cold oxygenated HBSS (Life Technologies, Grant Island, NY). After dissection of the tegmentrum vasculosum with fine microforceps, the remaining tissue is placed in 0.01% Type I collagenase (Sigma, St. Louis, MO) in HBSS for 3 minutes. The tectorial membrane is subsequently removed by grabbing it at the apical end of the basilar papilla (the avian equivalent of the organ of Corti) with microforceps and pulling it off the entire length of the sensory epithelium. Basilar papillae is fixed in 4% paraformaldehyde for 30 minutes, rinsed in PBS, and stored at 4°C until immunohistochemistry is performed.

### Immunochistochemistry of cochlear whole mounts

Immunochemical markers for progenitor cells and early differentiating hair cells are used to study hair cell loss and regeneration *in vivo* and in cultures. Two markers selectively label hair cells, calmodulin and TUJ1 β tubulin antibodies, and one marker selectively labels support cells, cytokeratin antibodies.

Briefly, whole mounts of the basilar papilla are treated with 0.5% H₂O₂ in PBS for 15 minutes to block endogenous peroxidase activity. After rinses with PBS 10% normal horse serum in 0.05% Triton X-100/PBS is added for 20 minutes to block nonspecific immunoglobulin (IgG) binding. For single labeling experiments, whole mounts are reacted with one of the following monoclonal antibodies for 2 hours at room temperature or overnight at 4°C: anti-Dictyastelium discoideum calmodulin (diluted 1:500, Sigma clone 6D4): TUJ1 (diluted 1:1000, a gift from T. Frankfurter, University of Virginia), which detects a neuron-specific class III β tubulin; anti-cytokeratins (diluted 1:200; clone 8.13, Sigma) raised against acidic and basic bovine epidermal keratins; or anti-cytokeratins (diluted 1:200; clones AF) and AE3, Boehringer Mannhein, Indianapolis, IN) raised against acidic and basic human epidermal keratins. Tissue is treated with biotinylated horse anti-mouse IgG (1:200; Vector Laboratories, Burlingame, CA) for 30 minutes followed by the avidin-biotin-horseradish peroxidase (HRP) reagent (ABC kit Ba-2000; Vector Laboratories). Up to this point, all rinse steps are performed in PBS. The tissue is transferred to 50 mM. Tris/HCl buffer (Tris, pH 7.6) and treated with 0.04% 3.3'-diaminobenzidine (DAB) and 0.05% H₂O₂ diluted in Tris for 3-10 minutes. The tissue is then rinsed once in Tris and left in Tris until embedding. For all immunoreactions, the primary antibody is omitted from the reactions as a negative control. Some immunoreacted whole mounts are mounted onto microscope slides coated with 9:1 glycerol/PBS. coverslipped, and examined with a Leitz Aristoplan microscope. Additional whole mounts are embedded in plastic as follows. Specimens are dehydrated through a graded series of ethanol washes (10 min each step) and placed in propylene oxide for two 10 minute intervals. Subsequently, whole mounts are placed in a 1:1 mixture of propylene oxide and soft Spurr's plastic, followed by two changes of 100% in a cassette mold overnight at 60°C to allow polymerization of the plastic. After they are embedded, immunoreacted whole mounts are sectioned at 3 µm in a basal-to-apical manner. Every fifth and sixth section throughout the basilar papilla is mounted onto a set of two chrome-alum subbed microscope slides. One set of sections is counterstained with toluidine blue. Both sets of sections are coverslipped with Permount (Fisher Scientific, Fair Lawn, NJ) and examined with a Leitz Aristoplan microscope.

For TUJI/calmodulin co-labeling, the TUJI antibody is detected in whole mounts using Bodipy/fluorsescin isothicyanate (FITC)-conjugated IgG (1:300; Molecular Probes, Eugene, OR), and the calmodulin antibody is detected in the same tissue with lissamine rhodamine conjugated IgG (1:300; Jackson lmmunoresearch Labs, Westgrover, PA). Whole mounts are mounted with Vectashield (Vector Labs) and examined with a BioRad MRC-1000 confocal laser scanning microscope. Images are digitized using Common Version 7 software (BioRad, Richmond, CA), imported into Photoshop (Adobe, Mountain View, Ca) and printed with a Phaser IISDX dye-sublimation printer (Tektronix, Beverton, OR).

### Differentiation of hair cells in vivo.

To assess the temporal and spatial progression of differentiation of regenerated hair cells, chicks are administered a single intramuscular injection of tritiated thymidine (³H-thymidine; 10 µ Ci/gm; 70-90 Ci/mmol) or an intraperitoneal injection of bromodeoxyuridine (BrdU; 100 mg/kg) at 3 d after gentamicin injection, when cell proliferation is maximal (Bhave, *et al*., 1995). Chicks are euthanized at 2, 24, 48, 72 or 240 hr (10 d) after injection of the S phase marker by an intraperitoncal injection of sodium pentobarbital. and whole mounts of the basilar papillae are removed and fixed. For chicks that received a BrdU injection, whole mounts are treated with 2N HCI in 0.05% Triton X-100 PBS for 15 minutes at 37°C. After several rinses in PBS, whole mounts are treated with 0.5% H₂O₂ in PBS for 15 minutes. Anti-BrdU monoclonal antibody (1:100: Becton Dickinson, San Jose. CA) is added for 1 hour and the antibody is detected via the ABC/HRP reaction described above. Brd-U-labeled whole mounts are then immunoreacted to detect calmodulin using the ABC/HRP reaction described above. For chicks that receive a ³H-thymidine injection, whole mounts are immunoreacted for calmodulin only. All basilar papillae are embedded in soft Spurr's resin and sectioned at 3 µm in a basal-to-apical manner, and serial sections are mounted onto chrom-alum subbed microscope slides. To detect ³H-thymidine, slides are dipped in NTB2 emulsion (1:1 dilution; Kodak), exposed at 4° for 5-14 days, developed in D-19, fixed, rinsed, and dried. For both proliferation labels, one set of sections is counterstained with toluidine blue and coverslipped with Permount.

### Quantification of 3H-thymidine/calmodulin labeling.

Basilar papillae are chosen for quantitative analysis of calmodulin and ³H-thymidine double labeling on the basis of their integrity after processing. Specifically, basilar papillae are included if they had intact cells in all regions and no overlying tectorial membrane, which may hamper penetration of the antibodies. Two to four basilar paillae are analyzed for each experimental treatment using a Leitz Aristoplan microscope.

To identify temporal and spatial patterns of cell proliferation and differentiation in basilar papillae damaged *in vivo*, two analyses are performed on calmodulin-immunoreacted basilar papillae from chicks that receive a single ³H-thymidine injection and are euthanized at different time intervals, as described above. First, the number of calmodulin-positive cells are counted with nuclei in either the lumenal compartment (corresponding to the lumenal one third of the epithelium) or the adlumenal compartment (corresponding to the adlumenal two thirds of the epithelium) from chicks at each survival time. The nuclear location and number of calmodulin-positive cells are determined in every section from one series of sections. This analysis is performed only on sections of the basal-most 300 µm of the epithelium. This analysis generates information about the rate of hair cell differentiation in aminoglycoside damaged regions. Second, the ³H-thymidine-positive/calmodulin negative cells and ³H-thymidine-positive/calmodulin-positive cells in the region are counted. This analysis provides data about the timing of support cell proliferation and support cell and hair cell differentiation in the basal region.

### Cochlear Epithelial Culture

For each experiment, eight chicks between 4 and 12 days posthatch are decapitated, and the cochlear ducts are removed through the round window and placed in ice-cold dissection medium composed of DMFM plus F12 (DMEM/F12; Life Technologies) and 1% bovine serum albumin (Sigma). The regmentum vascuolosum was removed, and the cochlear duct is placed in room temperature dissection medium with 0.01% collagenase Type 1 (Sigma) for 5 minutes. The tectorial membrane is dissected off the sensory epithelium, and the cochlear duct is left in collagenase for the sensory epithelium, and the cochlear duct is left in collagenase for an additional 10 minutes. The needle of tuberculin syringe is used to loosen the sensory epithelium from the basal lamina, generating large pieces of epithelium and leaving the underlying stroma, border cells, hyaline cells, and clear cells *in situ*. The isolated sensory epithelial cells, composed of hair cells and support cells, are transferred to culture composed of hair cells and support cells, are transferred to culture medium composed of DMEM/F12. 5% fetal bovine serum (Sigma), 2 mM sodium bicarbonate, 5 mM HEPES. 0.6% glucose and a hormone supplement [6 mM putrescine, 0.25 mg/ml insulin, 1 mg/ml transferring, 400 nM progesterone, and 3 µm sodium selenite (all from Sigma)], mildly titrated with a plastic 1 ml pipette, and spun at 500 rpm for 2 min. The resulting pellets are resuspended in 0.5 ml of culture medium and plated into one of the following: four wells of a 96-well plastic tissue culture plate (Nunc), four wells of a 16-well tissue culture chamber slide (Nunc), or two wells of a four-well plastic tissue culture plate containing 13 mm² glass coverslips. Coverslips and culture plates were either uncoated or coated with 3.0 µg/cm² laminin (Life Technologies) or 5 µg/cm² fibronectin (Boehringer Mannheim). All media were replenished every 3-4 d.

Hair cells in cultures are killed by adding 65 µM streptomycin (Life Technologies) to culture media at the time of plating. Some cultures are fixed after 2 days with paraformaldehyde for 15 minutes, and cell types in these cultures are characterized immunocytochemically using antibodies to calmodulin and cytokeratins (see below). Additional cultures are treated with 65 µM streptomycin for 5 d to eliminate all hair cells and are maintained an additional 5 days in control media. In some of these cultures, cells that begin to acquire a hair cell phenotype are detected using antibodies to calmodulin or TUJ1 (see below). To determine whether cells with a hair cell phenotype are generated in culture, additional cultures are provided with BrdU (1 µM) for the entire culture period, fixed with paraformaldehyde, and immunoreacted to detect BrdU and calmodulin (see below).

The number of mitotic support cells after different periods of culture are estimated using BrdU pulse/fix labeling. BrdU (10 µM; Sigma) is added to culture media for 2 hr at 2, 3, 5, 7, 9, or 11 d after plating. Immediately after the 2 hr, BrdU pulse cultures are fixed with paraformaldehyde and immunoreacted to detect BrdU (see below). Two culture wells per time point are analyzed.

The cell types that remained in the cochlear duct after sensory epithelia isolation and culturing are characterized as follows. After the sensory epithelium is dissected, the remaining tissue is fixed in 4% paraformaldehyde/0.1% glutaraldehyde overnight at 4°C, embedded in Spurr's plastic, and sectioned as described above for whole mounts. Sections are counterstained with toluidine blue.

### Immunohistochemistry of epithelial explants.

The types and dilutions of all primary antibodies used to characterize cultures are the same as those described above for whole mounts. The ABC/HRP method is used in all antibody reactions, except for BrdU/calmodulin double labeling (described below). For all immunoreactions, the primary antibody is omitted from some cultures as a negative control.

Long-term cultures supplemented with BrdU are double-labeled with antibodies to BrdU and calmodulin. First, BrdU is detected using a rat monoclonal antibody (Scar-Lab, Sussex, England) diluted at 1:200 overnight at 4°C followed by FITC-conjugated goat ant-rat antibody (Cappel) diluted at 1:400 for 45 min. Second, calmodulin is labeled in the same tissue using the HRP method described above for whole mounts.

Before microscopic analysis, the culture chambers are removed from slides, and explants are coverslipped with 9:1 glycerol/PBS. Cultures that are grown on coverslips are placed face up on top of a drop of glycerol on a microscope slide and coverslipped with additional glycerol. Labeled tissues are examined with a Leitz Aristopolan microscope. Cultures that are double-labeled for BrdU and calmodulin are examined using confocal microscopy.

### Quantification of pulse/fix BrdU labeling.

BrdU labeling is quantified in cultures as follows. The number of BrdU-labeled cells per square millimeter is determined using a 10 X 10 eyepiece reticule mounted into a Nikon NMS inverted microscope. The culture dish is arranged on the stage such that the top left boundary of the well was aligned with the top left reticule square. Using a 10X objective, the number of Brd-Unlabeled cells per reticule square is counted and recorded. The plate is then moved to the left until the reticule lined up with the next region to be analyzed. At the right-hand edge of the well, the plate is shifted upward, and counting proceeded from right to left across the well in the next row. Once counting had proceeded to the left-hand edge of the well, the plate is shifted upward again, and the process is restarted. The total number of labeled cells per well is then converted to the number of labeled cells per square millimeter. BrdU-labeled cells in at least three wells are counted for each time point.

### Filamentous actin labeling of hair cells.

Cultures or whole mounts of the basilar papilla are fixed with paraformaldehyde and labeled with rhodamine phalloidin (Molecular Probes) diluted 1:50 in 0.05% Triton X-100 in PBS for 1.5 hr. Cultures are coverslipped as described above, except with Vectashield (Vector Laboratories) rather than glycerol, and examined using epifluorescence microscopy.

The ototoxic effects of ototoxic antibiotics such as gentamicin are well characterized. For example, the addition of an ototoxic antibody to cultures causes complete hair cell loss by day 2 *in vitro* and generates cultures composed of calmodulin-negative, cytokeratin-positive support cells. *See* Stone, *et al., J. Neurosci. 16:* 6157-6174 (1996). OP-1, when provided prior to the administration of an ototoxic antibody, is expected to prevent loss of sensory hair cells, thereby preventing the damage induced by aminoglycoside toxicity and functional deficits resulting therefrom. When provided after the administration of an ototoxic antibody, OP-1 is expected to increase the number of sensory hair cells, thereby reversing the damage induced by aminoglycoside toxicity and functional deficits resulting therefrom.

### 10.2 Effects of Morphogen on Sensory Hair Cells in Mammalian Cochlear Epithelium After Cisplatin Toxicity

The animals used in this study are rats or guinea pigs. Sensory hair cell damage is induced by injecting animals with cisplatin (5 mg/kg, i.p.). Skilled artisans will understand and appreciate that sensory hair cells can also be damaged by exposure to an ototoxic aminoglycoside such as gentamicin [see above] or by ototoxic insult from resulting from exposure to frequency-specific otoacoustic emissions [Sie and Norton, *Otolaryngol. Head Neck Surg. 116*: 585-592 (1997), incorporated herein by reference].

In order to assess the effects of morphogens on sensory hair cells exposed to cisplatin, some animals are administered OP-1 intracochlearly. Before any manipulations, normal auditory function is assured by measuring auditory brainstem responses (ABR) to broad band click stimuli. Animals are then implanted with pumps filled with sterile solutions containing either OP-1 or vehicle. The animals were tested at intervals as described below and the experiments were terminated on day 13 after implantation. Then histological preparation and assessment was performed on all turns in both the treated and control ears.

### Intracochlear infusion of OP-1

Sterilized Alzet® osmotic pumps (model 2002) that infuse 0.5µl/h for 14 days (Alza Corp., Palo Alto, CA) are used for intracochlear infusion of OP-1 into the inner ear. The pumps are filled with the sterile OP-1 solution (230 µl/pump), and primed in a 37°C water bath for 4 h prior to implantation. Using a post auricular approach and sterile surgical techniques, a micro-cannula (178 µm diameter) is implanted into the scala tympani in the basal turn of the cochlea. The pump is attached to the cannula, and secured in a subcutaneous pocket between the scapulae as described previously (Brown, et *al.,* 1993). A hole is drilled through the skull at the vertex (1 cm posterior to the bregma, the intersection of the sagittal and coronal suture lines), into which a self-tapping stainless-steel screw is placed. The screw serves as an anchor for the cannula and as the active recording electrode for the ABR. Each animal is followed for 13 days.

In one group of animals, the prophylactic effects of morphogens on sensory hair cells exposed to ototoxic cisplatin are assessed by implanting an osmotic minipump containing OP-1 (0, 0.05, 0.5 or 5.0 µg/kg/day) 24 hours prior to the cisplatin injection. In another group of animals, the effects of morphogens on damaged sensory hair cells are assessed by implanting an osmotic minipump containing OP-1 (0, 0.05, 0.5 or 5.0 µg/kg/day) 1, 24, or 48 hours after the cisplatin injection.

### Auditory brain stem response measurements

Animals are anesthetized by administration of xylazine (10 mg/kg, IM) and ketamine (40 mg/kg, IM). In a sound-proof room, computer generated alternating polarity voltage pulses (160 µs duration, 50 pulses per s) are delivered through a transducer (Beyer B4-31.05.00) headphone element) positioned within the opening of the ear canal. Neurologic responses are collected by using the vertex screw as the epidural active recording electrode, a subdermal needle electrode placed at the mid-line of the skull approx. 2 cm anterior to bregma as a reference electrode, and subcutaneous needle electrode in the thigh as the ground. In animals whose threshold is 35 dB or greater, the contralateral ear is masked with 50 dB white noise during ABR collection. Approximately 1000 samples of 12.8 ms electrophysiological activity following stimulation are recorded. Stimuli are provided at various intensities to determine threshold, which is defined as the lowest stimulus intensity that produced a visually consistent waveform. ABR's are recorded on days 0, 1, 5, 8, and 13. At the completion of the experiment the animals are anesthetized, decapitated, and the ears are removed for histological evaluation.

### Preparation of epithelial cell cultures

At 13 days after the cisplatin injection, animals are euthanized by an intraperitoncal injection of sodium pentobarbital and then are decapitated. Cochlear ducts are removed and placed in ice-cold oxygenated HBSS (Life Technologies, Grant Island, NY). After dissection of the tegmentrum vasculosum with fine microforceps, the remaining tissue is placed in 0.01% Type I collagenase (Sigma, St. Louis, MO) in HBSS for 3 minutes. The tectorial membrane is subsequently removed by grabbing it at the apical end of the organ of Corti)with microforceps and pulling it off the entire length of the sensory epithelium. Basilar papillae is fixed in 4% paraformaldehyde for 30 minutes, rinsed in PBS, and stored at 4°C until immunohistochemistry is performed as described in Example 10.1.

OP-1, when provided prior to the administration of cisplatin, is expected to prevent loss of sensory hair cells, thereby preventing the damage induced by cisplatin toxicity and functional deficits resulting therefrom. When provided after the administration of cisplatin, OP-1 is expected to increase the number of sensory hair cells, thereby reversing the damage induced by cisplatin and restoring functional deficits resulting therefrom.

Similar routine modifications can be made in other accepted models of sensory perception, to confirm efficacy of morphogen treatment to alleviate or prevent loss of sensory hair cells and the resulting sensory perception deficits.

### SEQUENCE LISTING

(1) GENERAL INFORMATION:
   (i) APPLICANT: SAMPATH, KUBER T.
      RUEGER, David R.
      COHEN, Charles M.
      CHARETTE, Marc F.
      JIN, Donald F.
   (ii) TITLE OF INVENTION: MORPHOGEN-INDUCED REGENERATION OF SENSE PERCEPTION TISSUES
   (iii) NUMBER OF SEQUENCES: 9
   (iv) CORRESPONDENCE ADDRESS:
      (A) ADDRESSEE: PATENT ADMINISTRATOR, TESTA, HURWITZ & THIBEAULT, LLP
      (B) STREET: 125 HIGH STREET
      (C) CITY: BOSTON
      (D) STATE: MA
      (E) COUNTRY: USA
      (F) ZIP: 02110
   (v) COMPUTER READABLE FORM:
      (A) MEDIUM TYPE: Floppy disk
      (B) COMPUTER: IBM PC compatible
      (C) OPERATING SYSTEM: PC-DOS/MS-DOS
      (D) SOFTWARE: PatentIn Release #1.0, Version #1.30
   (vi) CURRENT APPLICATION DATA:
      (A) APPLICATION NUMBER:
      (B) FILING DATE:
      (C) CLASSIFICATION:
   (viii) ATTORNEY/AGENT INFORMATION:
      (A) NAME: PITCHER, EDMUND R.
      (B) REGISTRATION NUMBER: 27,829
      (C) REFERENCE/DOCKET NUMBER: CRP-095CPC
   (ix) TELECOMMUNICATION INFORMATION:
      (A) TELEPHONE: (617) 248-7000
      (B) TELEFAX: (617) 248-7100
(2) INFORMATION FOR SEQ ID NO:1:
   (i) SEQUENCE CHARACTERISTICS:
      (A) LENGTH: 1822 base pairs
      (B) TYPE: nucleic acid
      (C) STRANDEDNESS: single
      (D) TOPOLOGY: linear
   (ii) MOLECULE TYPE: cDNA
   (vi) ORIGINAL SOURCE:
      (A) ORGANISM: HOMO SAPIENS
      (F) TISSUE TYPE: HIPPOCAMPUS
   (ix) FEATURE:
      (A) NAME/KEY: CDS
      (B) LOCATION: 49..1341
      (C) IDENTIFICATION METHOD: experimental
      (D) OTHER INFORMATION: /function= "OSTEOGENIC PROTEIN"
         /product= "OP-1"
         /evidence= EXPERIMENTAL
         /standard_name= "OP-1"
   (xi) SEQUENCE DESCRIPTION: SEQ ID NO:1:
   GGTGCGGGCC CGGAGCCCGG AGCCCGGGTA GCGCGTAGAG CCGGCGCG ATG CAC GTG        57
                                                        Met His Val
                                                          1
   CGC TCA CTG CGA GCT GCG GCG CCG CAC AGC TTC GTG GCG CTC TGG GCA     105
   Arg Ser Leu Arg Ala Ala Ala Pro His Ser Phe Val Ala Leu Trp Ala
         5                  10                  15
   CCC CTG TTC CTG CTG CGC TCC GCC CTG GCC GAC TTC AGC CTG GAC AAC     153
   Pro Leu Phe Leu Leu Arg Ser Ala Leu Ala Asp Phe Ser Leu Asp Asn
    20                  25                  30                  35
   GAG GTG CAC TCG AGC TTC ATC CAC CGG CGC CTC CGC AGC CAG GAG CGG     201
   Glu Val His Ser Ser Phe Ile His Arg Arg Leu Arg Ser Gln Glu Arg
                    40                  45                  50
   CGG GAG ATG CAG CGC GAG ATC CTC TCC ATT TTG GGC TTG CCC CAC CGC     249
   Arg Glu Met Gln Arg Glu Ile Leu Ser Ile Leu Gly Leu Pro His Arg
                55                  60                  65
   CCG CGC CCG CAC CTC CAG GGC AAG CAC AAC TCG GCA CCC ATG TTC ATG     297
   Pro Arg Pro His Leu Gln Gly Lys His Asn Ser Ala Pro Met Phe Met
            70                  75                  80
   CTG GAC CTG TAC AAC GCC ATG GCG GTG GAG GAG GGC GGC GGG CCC GGC     345
   Leu Asp Leu Tyr Asn Ala Met Ala Val Glu Glu Gly Gly Gly Pro Gly
        85                  90                  95
   GGC CAG GGC TTC TCC TAC CCC TAC AAG GCC GTC TTC AGT ACC CAG GGC     393
   Gly Gln Gly Phe Ser Tyr Pro Tyr Lys Ala Val Phe Ser Thr Gln Gly
   100                 105                 110                 115
   CCC CCT CTG GCC AGC CTG CAA GAT AGC CAT TTC CTC ACC GAC GCC GAC     441
   Pro Pro Leu Ala Ser Leu Gln Asp Ser His Phe Leu Thr Asp Ala Asp
                   120                 125                 130
   ATG GTC ATG AGC TTC GTC AAC CTC GTG GAA CAT GAC AAG GAA TTC TTC     489
   Met Val Met Ser Phe Val Asn Leu Val Glu His Asp Lys Glu Phe Phe
               135                 140                 145
   CAC CCA CGC TAC CAC CAT CGA GAG TTC CGG TTT GAT CTT TCC AAG ATC     537
   His Pro Arg Tyr His His Arg Glu Phe Arg Phe Asp Leu Ser Lys Ile
           150                 155                 160
   CCA GAA GGG GAA GCT GTC ACG GCA GCC GAA TTC CGG ATC TAC AAG GAC     585
   Pro Glu Gly Glu Ala Val Thr Ala Ala Glu Phe Arg Ile Tyr Lys Asp
       165                 170                 175
   TAC ATC CGG GAA CGC TTC GAC AAT GAG ACG TTC CGG ATC AGC GTT TAT     633
   Tyr Ile Arg Glu Arg Phe Asp Asn Glu Thr Phe Arg Ile Ser Val Tyr
   180                 185                 190                 195
   CAG GTG CTC CAG GAG CAC TTG GGC AGG GAA TCG GAT CTC TTC CTG CTC     681
   Gln Val Leu Gln Glu His Leu Gly Arg Glu Ser Asp Leu Phe Leu Leu
                   200                 205                 210
   GAC AGC CGT ACC CTC TGG GCC TCG GAG GAG GGC TGG CTG GTG TTT GAC     729
   Asp Ser Arg Thr Leu Trp Ala Ser Glu Glu Gly Trp Leu Val Phe Asp
               215                 220                 225
   ATC ACA GCC ACC AGC AAC CAC TGG GTG GTC AAT CCG CGG CAC AAC CTG     777
   Ile Thr Ala Thr Ser Asn His Trp Val Val Asn Pro Arg His Asn Leu
           230                 235                 240
   GGC CTG CAG CTC TCG GTG GAG ACG CTG GAT GGG CAG AGC ATC AAC CCC     825
   Gly Leu Gln Leu Ser Val Glu Thr Leu Asp Gly Gln Ser Ile Asn Pro
       245                 250                 255
   AAG TTG GCG GGC CTG ATT GGG CGG CAC GGG CCC CAG AAC AAG CAG CCC     873
   Lys Leu Ala Gly Leu Ile Gly Arg His Gly Pro Gln Asn Lys Gln Pro
   260                 265                 270                 275
   TTC ATG GTG GCT TTC TTC AAG GCC ACG GAG GTC CAC TTC CGC AGC ATC     921
   Phe Met Val Ala Phe Phe Lys Ala Thr Glu Val His Phe Arg Ser Ile
                   280                 285                 290
   CGG TCC ACG GGG AGC AAA CAG CGC AGC CAG AAC CGC TCC AAG ACG CCC      969
   Arg Ser Thr Gly Ser Lys Gln Arg Ser Gln Asn Arg Ser Lys Thr Pro
               295                 300                 305
   AAG AAC CAG GAA GCC CTG CGG ATG GCC AAC GTG GCA GAG AAC AGC AGC     1017
   Lys Asn Gln Glu Ala Leu Arg Met Ala Asn Val Ala Glu Asn Ser Ser
           310                 315                 320
   AGC GAC CAG AGG CAG GCC TGT AAG AAG CAC GAG CTG TAT GTC AGC TTC     1065
   Ser Asp Gln Arg Gln Ala Cys Lys Lys His Glu Leu Tyr Val Ser Phe
       325                 330                 335
   CGA GAC CTG GGC TGG CAG GAC TGG ATC ATC GCG CCT GAA GGC TAC GCC     1113
   Arg Asp Leu Gly Trp Gln Asp Trp Ile Ile Ala Pro Glu Gly Tyr Ala
   340                 345                 350                 355
   GCC TAC TAC TGT GAG GGG GAG TGT GCC TTC CCT CTG AAC TCC TAC ATG     1161
   Ala Tyr Tyr Cys Glu Gly Glu Cys Ala Phe Pro Leu Asn Ser Tyr Met
                   360                 365                 370
   AAC GCC ACC AAC CAC GCC ATC GTG CAG ACG CTG GTC CAC TTC ATC AAC     1209
   Asn Ala Thr Asn His Ala Ile Val Gln Thr Leu Val His Phe Ile Asn
               375                 380                 385
   CCG GAA ACG GTG CCC AAG CCC TGC TGT GCG CCC ACG CAG CTC AAT GCC     1257
   Pro Glu Thr Val Pro Lys Pro Cys Cys Ala Pro Thr Gln Leu Asn Ala
           390                 395                 400
   ATC TCC GTC CTC TAC TTC GAT GAC AGC TCC AAC GTC ATC CTG AAG AAA     1305
   Ile Ser Val Leu Tyr Phe Asp Asp Ser Ser Asn Val Ile Leu Lys Lys
       405                 410                 415
   TAC AGA AAC ATG GTG GTC CGG GCC TGT GGC TGC CAC TAGCTCCTCC          1351
   Tyr Arg Asn Met Val Val Arg Ala Cys Gly Cys His
   420                 425                 430
   GAGAATTCAG ACCCTTTGGG GCCAAGTTTT TCTGGATCCT CCATTGCTCG CCTTGGCCAG   1411
   GAACCAGCAG ACCAACTGCC TTTTGTGAGA CCTTCCCCTC CCTATCCCCA ACTTTAAAGG   1471
   TGTGAGAGTA TTAGGAAACA TGAGCAGCAT ATGGCTTTTG ATCAGTTTTT CAGTGGCAGC   1531
   ATCCAATGAA CAAGATCCTA CAAGCTGTGC AGGCAAAACC TAGCAGGAAA AAAAAACAAC  1591
   GCATAAAGAA AAATGGCCGG GCCAGGTCAT TGGCTGGGAA GTCTCAGCCA TGCACGGACT   1651
   CGTTTCCAGA GGTAATTATG AGCGCCTACC AGCCAGGCCA CCCAGCCGTG GGAGGAAGGG   1711
   GGCGTGGCAA GGGGTGGGCA CATTGGTGTC TGTGCGAAAG GAAAATTGAC CCGGAAGTTC   1771
   CTGTAATAAA TGTCACAATA AAACGAATGA ATGAAAAAAA AAAAAAAAAA A            1822
(2) INFORMATION FOR SEQ ID NO:2:
   (i) SEQUENCE CHARACTERISTICS:
      (A) LENGTH: 431 amino acids
      (B) TYPE: amino acid
      (D) TOPOLOGY: linear
   (ii) MOLECULE TYPE: protein
   (xi) SEQUENCE DESCRIPTION: SEQ ID NO:2:
   Met His Val Arg Ser Leu Arg Ala Ala Ala Pro His Ser Phe Val Ala
   1 5 10 15
   Leu Trp Ala Pro Leu Phe Leu Leu Arg Ser Ala Leu Ala Asp Phe Ser
   20 25 30
   Leu Asp Asn Glu Val His Ser Ser Phe Ile His Arg Arg Leu Arg Ser
   35 40 45
   Gln Glu Arg Arg Glu Met Gln Arg Glu Ile Leu Ser Ile Leu Gly Leu
       50                  55                  60
   Pro His Arg Pro Arg Pro His Leu Gln Gly Lys His Asn Ser Ala Pro
   65                  70                  75                  80
   Met Phe Met Leu Asp Leu Tyr Asn Ala Met Ala Val Glu Glu Gly Gly
                   85                  90                  95
   Gly Pro Gly Gly Gln Gly Phe Ser Tyr Pro Tyr Lys Ala Val Phe Ser
               100                 105                 110
   Thr Gln Gly Pro Pro Leu Ala Ser Leu Gln Asp Ser His Phe Leu Thr
           115                 120                 125
   Asp Ala Asp Met Val Met Ser Phe Val Asn Leu Val Glu His Asp Lys
       130                 135                 140
   Glu Phe Phe His Pro Arg Tyr His His Arg Glu Phe Arg Phe Asp Leu
   145                 150                 155                 160
   Ser Lys Ile Pro Glu Gly Glu Ala Val Thr Ala Ala Glu Phe Arg Ile
                   165                 170                 175
   Tyr Lys Asp Tyr Ile Arg Glu Arg Phe Asp Asn Glu Thr Phe Arg Ile
               180                 185                 190
   Ser Val Tyr Gln Val Leu Gln Glu His Leu Gly Arg Glu Ser Asp Leu
           195                 200                 205
   Phe Leu Leu Asp Ser Arg Thr Leu Trp Ala Ser Glu Glu Gly Trp Leu
       210                 215                 220
   Val Phe Asp Ile Thr Ala Thr Ser Asn His Trp Val Val Asn Pro Arg
   225                 230                 235                 240
   His Asn Leu Gly Leu Gln Leu Ser Val Glu Thr Leu Asp Gly Gln Ser
                   245                 250                 255
   Ile Asn Pro Lys Leu Ala Gly Leu Ile Gly Arg His Gly Pro Gln Asn
               260                 265                 270
   Lys Gln Pro Phe Met Val Ala Phe Phe Lys Ala Thr Glu Val His Phe
           275                 280                 285
   Arg Ser Ile Arg Ser Thr Gly Ser Lys Gln Arg Ser Gln Asn Arg Ser
       290                 295                 300
   Lys Thr Pro Lys Asn Gln Glu Ala Leu Arg Met Ala Asn Val Ala Glu
   305                 310                 315                 320
   Asn Ser Ser Ser Asp Gln Arg Gln Ala Cys Lys Lys His Glu Leu Tyr
                   325                 330                 335
   Val Ser Phe Arg Asp Leu Gly Trp Gln Asp Trp Ile Ile Ala Pro Glu
               340                 345                 350
   Gly Tyr Ala Ala Tyr Tyr Cys Glu Gly Glu Cys Ala Phe Pro Leu Asn
           355                 360                 365
   Ser Tyr Met Asn Ala Thr Asn His Ala Ile Val Gln Thr Leu Val His
       370                 375                 380
   Phe Ile Asn Pro Glu Thr Val Pro Lys Pro Cys Cys Ala Pro Thr Gln
   385                 390                 395                 400
   Leu Asn Ala Ile Ser Val Leu Tyr Phe Asp Asp Ser Ser Asn Val Ile
                   405                 410                 415
   Leu Lys Lys Tyr Arg Asn Met Val Val Arg Ala Cys Gly Cys His
               420                 425                 430
(2) INFORMATION FOR SEQ ID NO:3:
   (i) SEQUENCE CHARACTERISTICS:
      (A) LENGTH: 102 amino acids
      (B) TYPE: amino acid
      (C) STRANDEDNESS:
      (D) TOPOLOGY: linear
   (ii) MOLECULE TYPE: protein
   (ix) FEATURE:
      (A) NAME/KEY: Protein
      (B) LOCATION: 1..102
      (D) OTHER INFORMATION: /label= OPX
         /note= "wherein each Xaa is independently selected from a group of one or more specified amino acids as defined in the specification."
   (xi) SEQUENCE DESCRIPTION: SEQ ID NO:3:
   Cys Xaa Xaa His Glu Leu Tyr Val Xaa Phe Xaa Asp Leu Gly Trp Xaa
   1               5                   10                  15
   Asp Trp Xaa Ile Ala Pro Xaa Gly Tyr Xaa Ala Tyr Tyr Cys Glu Gly
               20                  25                  30
   Glu Cys Xaa Phe Pro Leu Xaa Ser Xaa Met Asn Ala Thr Asn His Ala
           35                  40                  45
   Ile Xaa Gln Xaa Leu Val His Xaa Xaa Xaa Pro Xaa Xaa Val Pro Lys
       50                  55                  60
   Xaa Cys Cys Ala Pro Thr Xaa Leu Xaa Ala Xaa Ser Val Leu Tyr Xaa
   65                  70                  75                  80
   Asp Xaa Ser Xaa Asn Val Xaa Leu Xaa Lys Xaa Arg Asn Met Val Val
                   85                  90                  95
   Xaa Ala Cys Gly Cys His
               100
(2) INFORMATION FOR SEQ ID NO:4:
   (i) SEQUENCE CHARACTERISTICS:
      (A) LENGTH: 97 amino acids
      (B) TYPE: amino acid
      (C) STRANDEDNESS:
      (D) TOPOLOGY: linear
   (ii) MOLECULE TYPE: protein
   (ix) FEATURE:
      (A) NAME/KEY: Protein
      (B) LOCATION: 1..97
      (D) OTHER INFORMATION: /label= Generic-Seq-7
         /note= "wherein each Xaa is independently selected from a group of one or more specified amino acids as defined in the specification."
(2) INFORMATION FOR SEQ ID NO:5:
   (i) SEQUENCE CHARACTERISTICS:
      (A) LENGTH: 102 amino acids
      (B) TYPE: amino acid
      (C) STRANDEDNESS:
      (D) TOPOLOGY: linear
   (ii) MOLECULE TYPE: protein
   (ix) FEATURE:
      (A) NAME/KEY: protein
      (B) LOCATION: 1..102
      (D) OTHER INFORMATION: /label= Generic-Seq-8
         /note= "wherein each Xaa is independently selected from a group of one or more specified amino acids as defined in the specification."
   (xi) SEQUENCE DESCRIPTION: SEQ ID NO:5:
   Cys Xaa Xaa Xaa Xaa Leu Xaa Xaa Xaa Phe Xaa Xaa Xaa Gly Trp Xaa
   1               5                   10                  15
   Xaa Xaa Xaa Xaa Xaa Pro Xaa Xaa Xaa Xaa Ala Xaa Tyr Cys Xaa Gly
               20                  25                  30
   Xaa Cys Xaa Xaa Pro Xaa Xaa Xaa Xaa Xaa Xaa Xaa Xaa Asn His Ala
           35                  40                  45
   Xaa Xaa Xaa Xaa Xaa Xaa Xaa Xaa Xaa Xaa Xaa Xaa Xaa Xaa Xaa Xaa
       50                  55                  60
   Xaa Cys Cys Xaa Pro Xaa Xaa Xaa Xaa Xaa Xaa Xaa Xaa Leu Xaa Xaa
   65                  70                  75                  80
   Xaa Xaa Xaa Xaa Xaa Val Xaa Leu Xaa Xaa Xaa Xaa Xaa Met Xaa Val
                   85                  90                  95
   Xaa Xaa Cys Xaa Cys Xaa
               100
(2) INFORMATION FOR SEQ ID NO:6:
   (i) SEQUENCE CHARACTERISTICS:
      (A) LENGTH: 97 amino acids
      (B) TYPE: amino acid
      (C) STRANDEDNESS: single
      (D) TOPOLOGY: linear
   (ii) MOLECULE TYPE: protein
   (ix) FEATURE:
      (A) NAME/KEY: Protein
      (B) LOCATION: 1..97
      (D) OTHER INFORMATION: /label= Generic-Seq-9
         /note= "wherein each Xaa is independently selected from a group of one or more specified amino acids as defined in the specification."
   (xi) SEQUENCE DESCRIPTION: SEQ ID NO:6:
   Xaa Xaa Xaa Xaa Xaa Xaa Xaa Xaa Xaa Xaa Xaa Xaa Xaa Xaa Xaa Xaa
   1               5                   10                  15
   Pro Xaa Xaa Xaa Xaa Xaa Xaa Xaa Cys Xaa Gly Xaa Cys Xaa Xaa Xaa
               20                  25                  30
   Xaa Xaa Xaa Xaa Xaa Xaa Xaa Xaa Xaa Xaa Xaa Xaa Xaa Xaa Xaa Xaa
           35                  40                  45
   Xaa Xaa Xaa Xaa Xaa Xaa Xaa Xaa Xaa Xaa Xaa Xaa Xaa Cys Xaa Pro
       50                  55                  60
   Xaa Xaa Xaa Xaa Xaa Xaa Xaa Xaa Leu Xaa Xaa Xaa Xaa Xaa Xaa Xaa
   65                  70                  75                  80
   Xaa Xaa Xaa Xaa Xaa Xaa Xaa Xaa Xaa Xaa Xaa Xaa Xaa Cys Xaa Cys
                   85                  90                  95
   Xaa
(2) INFORMATION FOR SEQ ID NO:7:
   (i) SEQUENCE CHARACTERISTICS:
      (A) LENGTH: 102 amino acids
      (B) TYPE: amino acid
      (C) STRANDEDNESS: single
      (D) TOPOLOGY: linear
   (ii) MOLECULE TYPE: protein
   (ix) FEATURE:
      (A) NAME/KEY: Protein
      (B) LOCATION: 1..102
      (D) OTHER INFORMATION: /label= Generic-Seq-10
         /note= "wherein each Xaa is independently selected from a group of one or more specified amino acids as defined in the specification."
   (xi) SEQUENCE DESCRIPTION: SEQ ID NO:7:
   Cys Xaa Xaa Xaa Xaa Xaa Xaa Xaa Xaa Xaa Xaa Xaa Xaa Xaa Xaa Xaa
   1               5                   10                  15
   Xaa Xaa Xaa Xaa Xaa Pro Xaa Xaa Xaa Xaa Xaa Xaa Xaa Cys Xaa Gly
               20                  25                  30
   Xaa Cys Xaa Xaa Xaa Xaa Xaa Xaa Xaa Xaa Xaa Xaa Xaa Xaa Xaa Xaa
           35                  40                  45
   Xaa Xaa Xaa Xaa Xaa Xaa Xaa Xaa Xaa Xaa Xaa Xaa Xaa Xaa Xaa Xaa
       50                  55                  60
   Xaa Xaa Cys Xaa Pro Xaa Xaa Xaa Xaa Xaa Xaa Xaa Xaa Leu Xaa Xaa
   65                  70                  75                  80
   Xaa Xaa Xaa Xaa Xaa Xaa Xaa Xaa Xaa Xaa Xaa Xaa Xaa Xaa Xaa Xaa
                   85                  90                  95
   Xaa Xaa Cys Xaa Cys Xaa
               100
(2) INFORMATION FOR SEQ ID NO:8:
   (i) SEQUENCE CHARACTERISTICS:
      (A) LENGTH: 5 amino acids
      (B) TYPE: amino acid
      (C) STRANDEDNESS:
      (D) TOPOLOGY: linear
   (ii) MOLECULE TYPE: peptide
   (ix) FEATURE:
      (A) NAME/KEY: Protein
      (B) LOCATION: 1..5
      (D) OTHER INFORMATION: /note= "wherein each Xaa is independently selected from a group of one or more specified amino acids as defined in the specification"
   (xi) SEQUENCE DESCRIPTION: SEQ ID NO:8:
   Cys Xaa Xaa Xaa Xaa
   1               5
(2) INFORMATION FOR SEQ ID NO:9:
   (i) SEQUENCE CHARACTERISTICS:
      (A) LENGTH: 5 amino acids
      (B) TYPE: amino acid
      (C) STRANDEDNESS:
      (D) TOPOLOGY: linear
   (ii) MOLECULE TYPE: peptide
   (ix) FEATURE:
      (A) NAME/KEY: Protein
      (B) LOCATION: 1..5
      (D) OTHER INFORMATION: /note= "wherein each Xaa is independently selected from a group of one or more specified amino acids as defined in the specification"

## Claims

1. Use of a morphogen for the manufacture of a medicament for treatment of disorders of sense organs in a mammal to the exclusion of a sensory perception deficit following an ischemic or traumatic injury wherein the morphogen has an amino acid sequence selected from a sequence:
(a) having at least 70% homology with the C-terminal seven-cysteine skeleton of human OP-1, residues 330-431 of SEQ ID NO: 2;
(b) having greater than 60% amino acid sequence identity with said C-terminal seven-cysteine skeleton of human OP-1;
(c) defined by Generic Sequence 7, SEQ ID NO: 4;
(d) defined by Generic Sequence 8, SEQ ID NO: 5;
(e) defined by Generic Sequence 9, SEQ ID NO: 6;
(f) defined by Generic Sequence 10, SEQ ID NO: 7, and
(g) defined by OPX, SEQ ID NO: 3,
wherein said morphogen stimulates production of an N-CAM or L1 isoform by an NG108-15 cell *in vitro.*

2. Use of claim 1 wherein said sensory perception deficit is a loss of hearing, orientation due to vestibular dysfunction; olfaction; vision; taste or touch.

3. Use of a morphogen for the manufacture of a medicament for preserving sensory perception in a mammal to the exclusion of a sensory perception deficit following an ischemic or traumatic injury, wherein the morphogen has an amino acid sequence:
(a) having at least 70%homology with the C-terminal seven-cysteine skeleton of human OP-1, residues 330-431 of SEQ ID NO: 2;
(b) having greater than 60% amino acid sequence identity with said C-terminal seven-cysteine skeleton of human OP-1;
(c) defined by Generic Sequence 7, SEQ ID NO: 4;
(d) defined by Generic Sequence 8, SEQ ID NO: 5;
(e) defined by Generic Sequence 9, SEQ ID NO: 6;
(f) defined by Generic Sequence 10, SEQ ID NO: 7, and
(g) defined by OPX, SEQ ID NO: 3,
wherein said morphogen stimulates production of an N-CAM or L1 isoform by an NG108-15 cell *in vitro.*

4. Use of a morphogen for the manufacture of a medicament for restoring the integrity of a sensory perception tissue to the exclusion of a sensory perception deficit following an ischemic or traumatic injury, wherein the morphogen has an amino acid sequence:
(a) having at least 70%homology with the C-terminal seven-cysteine skeleton of human OP-1, residues 330-431 of SEQ ID NO: 2;
(b) having greater than 60% amino acid sequence identity with said C-terminal seven-cysteine skeleton of human OP-1;
(c) defined by Generic Sequence 7, SEQ ID NO: 4;
(d) defined by Generic Sequence 8, SEQ ID NO: 5;
(e) defined by Generic Sequence 9, SEQ ID NO: 6;
(f) defined by Generic Sequence 10, SEQ ID NO: 7, and
(g) defined by OPX, SEQ ID NO: 3,
wherein said morphogen stimulates production of an N-CAM or L1 isoform by an NG108-15 cell *in vitro*.

5. Use of a morphogen for the manufacture of a medicament for maintaining the sensory perception to the exclusion of a sensory perception deficit following an ischemic or traumatic injury, wherein the morphogen has an amino acid sequence:
(a) having at least 70%homology with the C-terminal seven-cysteine skeleton of human OP-1, residues 330-431 of SEQ ID NO: 2;
(b) having greater than 60% amino acid sequence identity with said C-terminal seven-cysteine skeleton of human OP-1;
(c) defined by Generic Sequence 7, SEQ ID NO: 4;
(d) defined by Generic Sequence 8, SEQ ID NO: 5;
(e) defined by Generic Sequence 9, SEQ ID NO: 6;
(f) defined by Generic Sequence 10, SEQ ID NO: 7, and
(g) defined by OPX, SEQ ID NO: 3,
wherein said morphogen stimulates production of an N-CAM or L1 isoform by an NG108-15 cell *in vitro*.

6. Use of a morphogen for the manufacture of a medicament for preventing degeneration of sensory perception tissue to the exclusion of a sensory perception deficit following an ischemic or traumatic injury, wherein the morphogen has an amino acid sequence:
(a) having at least 70%homology with the C-terminal seven-cysteine skeleton of human OP-1, residues 330-431 of SEQ ID NO: 2;
(b) having greater than 60% amino acid sequence identity with said C-terminal seven-cysteine skeleton of human OP-1;
(c) defined by Generic Sequence 7, SEQ ID NO: 4;
(d) defined by Generic Sequence 8, SEQ ID NO: 5;
(e) defined by Generic Sequence 9, SEQ ID NO: 6;
(f) defined by Generic Sequence 10, SEQ ID NO: 7, and
(g) defined by OPX, SEQ ID NO: 3,
wherein said morphogen stimulates production of an N-CAM or L1 isoform by an NG108-15 cell *in vitro.*

7. Use of a morphogen for the manufacture of a medicament for restoring vestibular function (for example in a mammal afflicted with a loss of hair cells) to the exclusion of a sensory perception deficit following an ischemic or traumatic injury, wherein the morphogen has an amino acid sequence:
(a) having at least 70%homology with the C-terminal seven-cysteine skeleton of human OP-1, residues 330-431 of SEQ ID NO: 2;
(b) having greater than 60% amino acid sequence identity with said C-terminal seven-cysteine skeleton of human OP-1;
(c) defined by Generic Sequence 7, SEQ ID NO: 4;
(d) defined by Generic Sequence 8, SEQ ID NO: 5;
(e) defined by Generic Sequence 9, SEQ ID NO: 6;
(f) defined by Generic Sequence 10, SEQ ID NO: 7, and
(g) defined by OPX, SEQ ID NO: 3,
wherein said morphogen stimulates production of an N-CAM or L1 isoform by an NG108-15 cell *in vitro.*

8. Use of a morphogen for the manufacture of a medicament for restoring hearing in a mammal afflicted with a loss of sensory hair cells to the exclusion of a sensory perception deficit following an ischemic or traumatic injury, wherein the morphogen has an amino acid sequence:
(a) having at least 70%homology with the C-terminal seven-cysteine skeleton of human OP-1, residues 330-431 of SEQ ID NO: 2;
(b) having greater than 60% amino acid sequence identity with said C-terminal seven-cysteine skeleton of human OP-1;
(c) defined by Generic Sequence 7, SEQ ID NO: 4;
(d) defined by Generic Sequence 8, SEQ ID NO: 5;
(e) defined by Generic Sequence 9, SEQ ID NO: 6;
(f) defined by Generic Sequence 10, SEQ ID NO: 7, and
(g) defined by OPX, SEQ ID NO: 3,
wherein said morphogen stimulates production of an N-CAM or L1 isoform by an NG108-15 cell *in vitro.*

9. Use of a morphogen for the manufacture of a medicament for restoring the integrity of a sensory hair cells to the exclusion of a sensory perception deficit following an ischemic or traumatic injury, wherein the morphogen has amino acid sequence selected from the group consisting of a sequence:
(a) having at least 70%homology with the C-terminal seven-cysteine skeleton of human OP-1, residues 330-431 of SEQ ID NO: 2;
(b) having greater than 60% amino acid sequence identity with said C-terminal seven-cysteine skeleton of human OP-1;
(c) defined by Generic Sequence 7, SEQ ID NO: 4;
(d) defined by Generic Sequence 8, SEQ ID NO: 5;
(e) defined by Generic Sequence 9, SEQ ID NO: 6;
(f) defined by Generic Sequence 10, SEQ ID NO: 7, and
(g) defined by OPX, SEQ ID NO: 3,
wherein said morphogen stimulates production of an N-CAM or L1 isoform by an NG108-15 cell *in vitro.*

10. Use of a morphogen for the manufacture of a medicament for preventing degeneration of sensory hair cells to the exclusion of a sensory perception deficit following an ischemic or traumatic injury, wherein the morphogen has an amino acid sequence:
(a) having at least 70%homology with the C-terminal seven-cysteine skeleton of human OP-1, residues 330-431 of SEQ ID NO: 2;
(b) having greater than 60% amino acid sequence identity with said C-terminal seven-cysteine skeleton of human OP-1;
(c) defined by Generic Sequence 7, SEQ ID NO: 4;
(d) defined by Generic Sequence 8, SEQ ID NO: 5;
(e) defined by Generic Sequence 9, SEQ ID NO: 6;
(f) defined by Generic Sequence 10, SEQ ID NO: 7, and
(g) defined by OPX, SEQ ID NO: 3,
wherein said morphogen stimulates production of an N-CAM or L1 isoform by an NG108-15 cell *in vitro.*

11. Use of a morphogen for the manufacture of a medicament for promoting growth of sensory hair cells to the exclusion of a sensory perception deficit following an ischemic or traumatic injury, wherein the morphogen has an amino acid sequence:
(a) having at least 70%homology with the C-terminal seven-cysteine skeleton of human OP-1, residues 330-431 of SEQ ID NO: 2;
(b) having greater than 60% amino acid sequence identity with said C-terminal seven-cysteine skeleton of human OP-1;
(c) defined by Generic Sequence 7, SEQ ID NO: 4;
(d) defined by Generic Sequence 8, SEQ ID NO: 5;
(e) defined by Generic Sequence 9, SEQ ID NO: 6;
(f) defined by Generic Sequence 10, SEQ ID NO: 7, and
(g) defined by OPX, SEQ ID NO: 3,
wherein said morphogen stimulates production of an N-CAM or L1 isoform by an NG108-15 cell *in vitro.*

12. The use of any one of the preceding claims, wherein said morphogen is OP-1.

13. The use of any one of claims 1-11, wherein said morphogen is selected from the group consisting of human OP-2, mouse OP-2, 60A, GDF-1, BMP2A, BMP2B, DPP, Vgl, Vgr-1, BMP3, BMP5, and BMP6, wherein said morphogen stimulates production of an N-CAM or L1 isoform by an NG108-15 cell *in vitro.*

14. The use of any one of the preceding claims wherein said morphogen is non-covalently complexed with at least one morphogen pro domain.

## Patentansprüche

1. Verwendung eines Morphogens zur Herstellung eines Medikamentes zur Behandlung von Störungen von Sinnesorganen in einem Säugetier unter Ausschluss eines Sinneswahrnehmungsdefizits im Anschluss an eine ischämische oder traumatische Verletzung, wobei das Morphogen eine Aminosäuresequenz aufweist, die aus einer Sequenz ausgewählt ist:
(a) die zumindest 70% Homologie mit dem C-terminalen Sieben-Cystein-Skelett von humanem OP-1, Reste 330-431 von Sequenz ID NO: 2 aufweist;
(b) die mehr als 60% Aminosäuresequenzidentität mit dem C-terminalen Sieben-Cystein-Skelett von humanem OP-1 aufweist;
(c) die durch die generische Sequenz 7, Sequenz ID NO: 4 definiert ist;
(d) die durch die generische Sequenz 8, Sequenz ID NO: 5 definiert ist;
(e) die durch die generische Sequenz 9, Sequenz ID NO: 6 definiert ist;
(f) die durch die generische Sequenz 10, Sequenz ID NO: 7 definiert ist, und
(g) die durch OPX, Sequenz ID NO: 3 definiert ist,
wobei das Morphogen die Produktion einer N-CAM- oder L1-Isoform durch eine NG108-15-Zelle in vitro stimuliert.

2. Verwendung nach Anspruch 1, wobei das Sinneswahrnehmungsdefizit ein Verlust des Hörens, der Orientierung aufgrund einer Vestibularisstörung; des Geruchs; der Sehfähigkeit; des Geschmacks oder des Tastsinnes ist.

3. Verwendung eines Morphogens zur Herstellung eines Medikamentes zur Erhaltung der Sinneswahrnehmung in einem Säugetier unter Ausschluss eines Sinneswahrnehmungsdefizits im Anschluss an eine ischämische oder traumatische Verletzung, wobei das Morphogen eine Aminosäuresequenz aufweist:
(a) die zumindest 70% Homologie mit dem C-terminalen Sieben-Cystein-Skelett von humanem OP-1, Reste 330-431 von Sequenz ID NO: 2 aufweist;
(b) die mehr als 60% Aminosäuresequenzidentität mit dem C-terminalen Sieben-Cystein-Skelett von humanem OP-1 aufweist;
(c) die durch die generische Sequenz 7, Sequenz ID NO: 4 definiert ist;
(d) die durch die generische Sequenz 8, Sequenz ID NO: 5 definiert ist;
(e) die durch die generische Sequenz 9, Sequenz ID NO: 6 definiert ist;
(f) die durch die generische Sequenz 10, Sequenz ID NO: 7 definiert ist, und
(g) die durch OPX, Sequenz ID NO: 3 definiert ist,
wobei das Morphogen die Produktion einer N-CAM- oder L1-Isoform durch eine NG108-15-Zelle in vitro stimuliert.

4. Verwendung eines Morphogens zur Herstellung eines Medikamentes zur Wiederherstellung der Integrität eines Sinneswahrnehmungsgewebes unter Ausschluss eines Sinneswahrnehmungsdefizits im Anschluss an eine ischämische oder traumatische Verletzung, wobei das Morphogen eine Aminosäuresequenz aufweist:
(a) die zumindest 70% Homologie mit dem C-terminalen Sieben-Cystein-Skelett von humanem OP-1, Reste 330-431 von Sequenz ID NO: 2 ausweist;
(b) die mehr als 60% Aminosäuresequenzidentität mit dem C-terminalen Sieben-Cystein-Skelett von humanem OP-1 aufweist;
(c) die durch die generische Sequenz 7, Sequenz ID NO: 4 definiert ist;
(d) die durch die generische Sequenz 8, Sequenz ID NO: 5 definiert ist;
(e) die durch die generische Sequenz 9, Sequenz ID NO: 6 definiert ist;
(f) die durch die generische Sequenz 10, Sequenz ID NO: 7 definiert ist, und
(g) die durch OPX, Sequenz ID NO: 3 definiert ist,
wobei das Morphogen die Produktion einer N-CAM- oder L1-Isoform durch eine NG108-15-Zelle in vitro stimuliert.

5. Verwendung eines Morphogens zur Herstellung eines Medikamentes zur Aufrechterhaltung der Sinneswahrnehmung unter Ausschluss eines Sinneswahrnehmungsdefizits im Anschluss an eine ischämische oder traumatische Verletzung, wobei das Morphogen eine Aminosäuresequenz aufweist:
(a) die zumindest 70% Homologie mit dem C-terminalen Sieben-Cystein-Skelett von humanem OP-1, Reste 330-431 von Sequenz ID NO: 2 aufweist;
(b) die mehr als 60% Aminosäuresequenzidentität mit dem C-terminalen Sieben-Cystein-Skelett von humanem OP-1 aufweist;
(c) die durch die generische Sequenz 7, Sequenz ID NO: 4 definiert ist;
(d) die durch die generische Sequenz 8, Sequenz ID NO: 5 definiert ist;
(e) die durch die generische Sequenz 9, Sequenz ID NO: 6 definiert ist;
(f) die durch die generische Sequenz 10, Sequenz ID NO: 7 definiert ist, und
(g) die durch OPX, Sequenz ID NO: 3 definiert ist,
wobei das Morphogen die Produktion einer N-CAM- oder L1-Isoform durch eine NG108-15-Zelle in vitro stimuliert.

6. Verwendung eines Morphogens zur Herstellung eines Medikamentes zur Vorbeugung der Degeneration eines Sinneswahrnehmungsgewebes unter Ausschluss eines Sinneswahrnehmungsdefizits im Anschluss an eine ischämische oder traumatische Verletzung, wobei das Morphogen eine Aminosäuresequenz aufweist:
(a) die zumindest 70% Homologie mit dem C-terminalen Sieben-Cystein-Skelett von humanem OP-1, Reste 330-431 von Sequenz ID NO: 2 aufweist;
(b) die mehr als 60% Aminosäuresequenzidentität mit dem C-terminalen Sieben-Cystein-Skelett von humanem OP-1 aufweist;
(c) die durch die generische Sequenz 7, Sequenz ID NO: 4 definiert ist;
(d) die durch die generische Sequenz 8, Sequenz ID NO: 5 definiert ist;
(e) die durch die generische Sequenz 9, Sequenz ID NO: 6 definiert ist;
(f) die durch die generische Sequenz 10, Sequenz ID NO: 7 definiert ist, und
(g) die durch OPX, Sequenz ID NO: 3 definiert ist,
wobei das Morphogen die Produktion einer N-CAM- oder L1-Isoform durch eine NG108-15-Zelle in vitro stimuliert.

7. Verwendung eines Morphogens zur Herstellung eines Medikamentes zur Wiederherstellung der Vestibularfunktion (beispielsweise bei einem Säugetier, das von einem Verlust von Haarzellen betroffen ist) unter Ausschluss eines Sinneswahrnehmungsdefizits im Anschluss an eine ischämische oder traumatische Verletzung, wobei das Morphogen eine Aminosäuresequenz aufweist:
(a) die zumindest 70% Homologie mit dem C-terminalen Sieben-Cystein-Skelett von humanem OP-1, Reste 330-431 von Sequenz ID NO: 2 aufweist;
(b) die mehr als 60% Aminosäuresequenzidentität mit dem C-terminalen Sieben-Cystein-Skelett von humanem OP-1 aufweist;
(c) die durch die generische Sequenz 7, Sequenz ID NO: 4 definiert ist;
(d) die durch die generische Sequenz 8, Sequenz ID NO: 5 definiert ist;
(e) die durch die generische Sequenz 9, Sequenz ID NO: 6 definiert ist;
(f) die durch die generische Sequenz 10, Sequenz ID NO: 7 definiert ist, und
(g) die durch OPX, Sequenz ID NO: 3 definiert ist,
wobei das Morphogen die Produktion einer N-CAM- oder L1-Isoform durch eine NG108-15-Zelle in vitro stimuliert.

8. Verwendung eines Morphogens zur Herstellung eines Medikamentes zur Wiederherstellung des Hörvermögens bei einem Säugetier, das von einem Verlust von Sinneshaarzellen betroffen ist, unter Ausschluss eines Sinneswahrnehmungsdefizits im Anschluss an eine ischämische oder traumatische Verletzung, wobei das Morphogen eine Aminosäuresequenz aufweist:
(a) die zumindest 70% Homologie mit dem C-terminalen Sieben-Cystein-Skelett von humanem OP-1, Reste 330-431 von Sequenz ID NO: 2 aufweist;
(b) die mehr als 60% Aminosäuresequenzidentität mit dem C-terminalen Sieben-Cystein-Skelett von humanem OP-1 aufweist;
(c) die durch die generische Sequenz 7, Sequenz ID NO: 4 definiert ist;
(d) die durch die generische Sequenz 8, Sequenz ID NO: 5 definiert ist;
(e) die durch die generische Sequenz 9, Sequenz ID NO: 6 definiert ist;
(f) die durch die generische Sequenz 10, Sequenz ID NO: 7 definiert ist, und
(g) die durch OPX, Sequenz ID NO: 3 definiert ist,
wobei das Morphogen die Produktion einer N-CAM- oder L1-Isoform durch eine NG108-15-Zelle in vitro stimuliert.

9. Verwendung eines Morphogens zur Herstellung eines Medikamentes zur Wiederherstellung der Integrität von Sinneshaarzellen unter Ausschluss eines Sinneswahrnehmungsdefizits im Anschluss an eine ischämische oder traumatische Verletzung, wobei das Morphogen eine Aminosäuresequenz ausgewählt aus der Gruppe aufweist, die aus einer Sequenz besteht:
(a) die zumindest 70% Homologie mit dem C-terminalen Sieben-Cystein-Skelett von humanem OP-1, Reste 330-431 von Sequenz ID NO: 2 aufweist;
(b) die mehr als 60% Aminosäuresequenzidentität mit dem C-terminalen Sieben-Cystein-Skelett von humanem OP-1 aufweist;
(c) die durch die generische Sequenz 7, Sequenz ID NO: 4 definiert ist;
(d) die durch die generische Sequenz 8, Sequenz ID NO: 5 definiert ist;
(e) die durch die gecerische Sequenz 9, Sequenz ID NO: 6 definiert ist;
(f) die durch die generische Sequenz 10, Sequenz ID NO: 7 definiert ist, und
(g) die durch OPX, Sequenz ID NO: 3 definiert ist,
wobei das Morphogen die Produktion einer N-CAM- oder L1-Isoform durch eine NG108-15-Zelle in vitro stimuliert.

10. Verwendung eines Morphogens zur Herstellung eines Medikamentes zur Vorbeugung einer Degeneration von Sinneshaarzellen unter Ausschluss eines Sinneswahrnehmungsdefizits im Anschluss an eine ischämische oder traumatische Verletzung, wobei das Morphogen eine Aminosäuresequenz aufweist:
(a) die zumindest 70% Homologie mit dem C-terminalen Sieben-Cystein-Skelett von humanem OP-1, Reste 330-431 von Sequenz ID NO: 2 aufweist;
(b) die mehr als 60% Aminosäuresequenzidentität mit dem C-terminalen Sieben-Cystein-Skelett von humanem OP-1 aufweist;
(c) die durch die generische Sequenz 7, Sequenz ID NO: 4 definiert ist;
(d) die durch die generische Sequenz 8, Sequenz ID NO: 5 definiert ist;
(e) die durch die generische Sequenz 9, Sequenz ID NO: 6 definiert ist;
(f) die durch die generische Sequenz 10, Sequenz ID NO: 7 definiert ist, und
(g) die durch OPX, Sequenz ID NO: 3 definiert ist,
wobei das Morphogen die Produktion einer N-CAM- oder L1-Isoform durch eine NG108-15-Zelle in vitro stimuliert.

11. Verwendung eines Morphogens zur Herstellung eines Medikamentes zur Förderung des Wachstums von Sinneshaarzellen unter Ausschluss eines Sinneswahrnehmungsdefizits im Anschluss an eine ischämische oder traumatische Verletzung, wobei das Morphogen eine Aminosäuresequenz aufweist:
(a) die zumindest 70% Homologie mit dem C-terminalen Sieben-Cystein-Skelett von humanem OP-1, Reste 330-431 von Sequenz ID NO: 2 aufweist;
(b) die mehr als 60% Aminosäuresequenzidentität mit dem C-terminalen Sieben-Cystein-Skelett von humanem OP-1 aufweist;
(c) die durch die generische Sequenz 7, Sequenz ID NO: 4 definiert ist;
(d) die durch die generische Sequenz 8, Sequenz ID NO: 5 definiert ist;
(e) die durch die generische Sequenz 9, Sequenz ID NO: 6 definiert ist;
(f) die durch die generische Sequenz 10, Sequenz ID NO: 7 definiert ist, und
(g) die durch OPX, Sequenz ID NO: 3 definiert ist,
wobei das Morphogen die Produktion einer N-CAM- oder L1-Isoform durch eine NG108-15-Zelle in vitro stimuliert.

12. Verwendung nach einem der vorhergehenden Ansprüche, wobei das Morphogen OP-1 ist.

13. Verwendung nach einem der Ansprüche 1-11, wobei das Morphogen aus der Gruppe ausgewählt ist, die aus humanem OP-2, Maus OP-2, 60A, GDF-1, BMP2A, BMP2B, DPP, Vgl, Vgr-1, BMP3, BMP5 und BMP6 besteht, wobei das Morphogen die Produktion einer N-CAM- oder L1-Isoform durch eine NG108-15-Zelle in vitro stimuliert.

14. Verwendung nach einem der vorhergehenden Ansprüche, wobei das Morphogen mit zumindest einem Morphogen pro Domäne nicht-kovalent komplexiert ist.

## Revendications

1. Utilisation d'un morphogène pour la préparation d'un médicament destiné au traitement de troubles des organes sensoriels chez un mammifère, à l'exclusion d'une régression de la perception sensorielle suite à une ischémie ou à un traumatisme, dans laquelle le morphogène possède une séquence d'acides aminés choisie parmi le groupe de séquences comprenant :
(a) une séquence possédant une homologie à concurrence d'au moins 70 % avec le squelette à extrémité C-terminale comportant sept résidus cystéine de la OP-1 humaine, les résidus 330 - 431 de SEQ ID N0 : 2 ;
(b) une séquence possédant une identité de séquence à concurrence de plus de 60 % avec ledit squelette à extrémité C-terminale comportant sept résidus cystéine de la OP-1 humaine ;
(c) une séquence définie par la Séquence Générique 7, SEQ ID N0 : 4 ;
(d) une séquence définie par la Séquence Générique 8, SEQ ID N0 : 5 ;
(e) une séquence définie par la Séquence Générique 9, SEQ ID N0 : 6 ;
(f) une séquence définie par la Séquence Générique 10, SEQ ID N0 : 7 ; et
(g) une séquence définie par OPX, SEQ ID N0 : 3,
dans laquelle ledit morphogène stimule la production d'une isoforme de N-CAM ou de L1 via une cellule NG108-15 *in vitro*.

2. Utilisation selon la revendication 1, dans laquelle ladite régression de la perception sensorielle se manifeste par une perte de l'audition, de l'orientation due à un dysfonctionnement vestibulaire, de l'olfaction, de la vision, du goût ou du toucher.

3. Utilisation d'un morphogène pour la préparation d'un médicament destiné à préserver la perception sensorielle chez un mammifère, à l'exclusion d'une régression de la perception sensorielle suite à une ischémie ou à un traumatisme, dans laquelle le morphogène possède une séquence d'acides aminés choisie parmi le groupe de séquences comprenant :
(a) une séquence possédant une homologie à concurrence d'au moins 70 % avec le squelette à extrémité C-terminale comportant sept résidus cystéine de la OP-1 humaine, les résidus 330 - 431 de SEQ ID N0 : 2 ;
(b) une séquence possédant une identité de séquence à concurrence de plus de 60 % avec ledit squelette à extrémité C-terminale comportant sept résidus cystéine de la OP-1 humaine ;
(c) une séquence définie par la Séquence Générique 7, SEQ ID N0 : 4 ;
(d) une séquence définie par la Séquence Générique 8, SEQ ID N0 : 5 ;
(e) une séquence définie par la Séquence Générique 9, SEQ ID N0 : 6 ;
(f) une séquence définie par la Séquence Générique 10, SEQ ID N0 : 7 ; et
(g) une séquence définie par OPX, SEQ ID N0 : 3,
dans laquelle ledit morphogène stimule la production d'une isoforme de N-CAM ou de L1 via une cellule NG108-15 *in vitro*.

4. Utilisation d'un morphogène pour la préparation d'un médicament destiné à restaurer l'intégrité d'un tissu de perception sensorielle, à l'exclusion d'une régression de la perception sensorielle suite à une ischémie ou à un traumatisme, dans laquelle le morphogène possède une séquence d'acides aminés choisie parmi le groupe de séquences comprenant :
(a) une séquence possédant une homologie à concurrence d'au moins 70 % avec le squelette à extrémité C-terminale comportant sept résidus cystéine de la OP-1 humaine, les résidus 330 - 431 de SEQ ID N0 : 2 ;
(b) une séquence possédant une identité de séquence à concurrence de plus de 60 % avec ledit squelette à extrémité C-terminale comportant sept résidus cystéine de la OP-1 humaine ;
(c) une séquence définie par la Séquence Générique 7, SEQ ID N0 : 4 ;
(d) une séquence définie par la Séquence Générique 8, SEQ ID N0 : 5 ;
(e) une séquence définie par la Séquence Générique 9, SEQ ID N0 : 6 ;
(f) une séquence définie par la Séquence Générique 10, SEQ ID N0 : 7 ; et
(g) une séquence définie par OPX, SEQ ID N0 : 3,
dans laquelle ledit morphogène stimule la production d'une isoforme de N-CAM ou de L1 via une cellule NG108-15 *in vitro*.

5. Utilisation d'un morphogène pour la préparation d'un médicament destiné à maintenir la perception sensorielle, à l'exclusion d'une régression de la perception sensorielle suite à une ischémie ou à un traumatisme, dans laquelle le morphogène possède une séquence d'acides aminés choisie parmi le groupe de séquences comprenant :
(a) une séquence possédant une homologie à concurrence d'au moins 70 % avec le squelette à extrémité C-terminale comportant sept résidus cystéine de la OP-1 humaine, les résidus 330 - 431 de SEQ ID N0 : 2 ;
(b) une séquence possédant une identité de séquence à concurrence de plus de 60 % avec ledit squelette à extrémité C-terminale comportant sept résidus cystéine de la OP-1 humaine ;
(c) une séquence définie par la Séquence Générique 7, SEQ ID N0 : 4 ;
(d) une séquence définie par la Séquence Générique 8, SEQ ID N0 : 5 ;
(e) une séquence définie par la Séquence Générique 9, SEQ ID N0 : 6 ;
(f) une séquence définie par la Séquence Générique 10, SEQ ID N0 : 7 ; et
(g) une séquence définie par OPX, SEQ ID N0 : 3,
dans laquelle ledit morphogène stimule la production d'une isoforme de N-CAM ou de L1 via une cellule NG108-15 *in vitro*.

6. Utilisation d'un morphogène pour la préparation d'un médicament destiné à empêcher la dégénérescence d'un tissu de perception sensorielle, à l'exclusion d'une régression de la perception sensorielle suite à une ischémie ou à un traumatisme, dans laquelle le morphogène possède une séquence d'acides aminés choisie parmi le groupe de séquences comprenant :
(a) une séquence possédant une homologie à concurrence d'au moins 70 % avec le squelette à extrémité C-terminale comportant sept résidus cystéine de la OP-1 humaine, les résidus 330 - 431 de SEQ ID N0 : 2 ;
(b) une séquence possédant une identité de séquence à concurrence de plus de 60 % avec ledit squelette à extrémité C-terminale comportant sept résidus cystéine de la OP-1 humaine ;
(c) une séquence définie par la Séquence Générique 7, SEQ ID N0 : 4 ;
(d) une séquence définie par la Séquence Générique 8, SEQ ID N0 : 5 ;
(e) une séquence définie par la Séquence Générique 9, SEQ ID N0 : 6 ;
(f) une séquence définie par la Séquence Générique 10, SEQ ID N0 : 7 ; et
(g) une séquence définie par OPX, SEQ ID N0 : 3,
dans laquelle ledit morphogène stimule la production d'une isoforme de N-CAM ou de L1 via une cellule NG108-15 *in vitro*.

7. Utilisation d'un morphogène pour la préparation d'un médicament destiné à restaurer la fonction vestibulaire (par exemple chez un mammifère souffrant d'une perte de cellules épithéliales pourvues de poils), à l'exclusion d'une régression de la perception sensorielle suite à une ischémie ou à un traumatisme, dans laquelle le morphogène possède une séquence d'acides aminés choisie parmi le groupe de séquences comprenant :
(a) une séquence possédant une homologie à concurrence d'au moins 70 % avec le squelette à extrémité C-terminale comportant sept résidus cystéine de la OP-1 humaine, les résidus 330 - 431 de SEQ ID N0 : 2 ;
(b) une séquence possédant une identité de séquence à concurrence de plus de 60 % avec ledit squelette à extrémité C-terminale comportant sept résidus cystéine de la OP-1 humaine ;
(c) une séquence définie par la Séquence Générique 7, SEQ ID N0 : 4 ;
(d) une séquence définie par la Séquence Générique 8, SEQ ID N0 : 5 ;
(e) une séquence définie par la Séquence Générique 9, SEQ ID N0 : 6 ;
(f) une séquence définie par la Séquence Générique 10, SEQ ID N0 : 7 ; et
(g) une séquence définie par OPX, SEQ ID N0 : 3,
dans laquelle ledit morphogène stimule la production d'une isoforme de N-CAM ou de L1 via une cellule NG108-15 *in vitro*.

8. Utilisation d'un morphogène pour la préparation d'un médicament destiné à restaurer l'audition chez un mammifère souffrant d'une perte de cellules épithéliales pourvues de poils, à l'exclusion d'une régression de la perception sensorielle suite à une ischémie ou à un traumatisme, dans laquelle le morphogène possède une séquence d'acides aminés choisie parmi le groupe de séquences comprenant :
(a) une séquence possédant une homologie à concurrence d'au moins 70 % avec le squelette à extrémité C-terminale comportant sept résidus cystéine de la OP-1 humaine, les résidus 330 - 431 de SEQ ID N0 : 2 ;
(b) une séquence possédant une identité de séquence à concurrence de plus de 60 % avec ledit squelette à extrémité C-terminale comportant sept résidus cystéine de la OP-1 humaine ;
(c) une séquence définie par la Séquence Générique 7, SEQ ID N0 : 4 ;
(d) une séquence définie par la Séquence Générique 8, SEQ ID N0 : 5 ;
(e) une séquence définie par la Séquence Générique 9, SEQ ID N0 : 6 ;
(f) une séquence définie par la Séquence Générique 10, SEQ ID N0 : 7 ; et
(g) une séquence définie par OPX, SEQ ID N0 : 3,
dans laquelle ledit morphogène stimule la production d'une isoforme de N-CAM ou de L1 via une cellule NG108-15 *in vitro*.

9. Utilisation d'un morphogène pour la préparation d'un médicament destiné à restaurer l'intégrité des cellules épithéliales pourvues de poils, à l'exclusion d'une régression de la perception sensorielle suite à une ischémie ou à un traumatisme, dans laquelle le morphogène possède une séquence d'acides aminés choisie parmi le groupe de séquences comprenant :
(a) une séquence possédant une homologie à concurrence d'au moins 70 % avec le squelette à extrémité C-terminale comportant sept résidus cystéine de la OP-1 humaine, les résidus 330 - 431 de SEQ ID N0 : 2 ;
(b) une séquence possédant une identité de séquence à concurrence de plus de 60 % avec ledit squelette à extrémité C-terminale comportant sept résidus cystéine de la OP-1 humaine ;
(c) une séquence définie par la Séquence Générique 7, SEQ ID N0 : 4 ;
(d) une séquence définie par la Séquence Générique 8, SEQ ID N0 : 5 ;
(e) une séquence définie par la Séquence Générique 9, SEQ ID N0 : 6 ;
(f) une séquence définie par la Séquence Générique 10, SEQ ID N0 : 7 ; et
(g) une séquence définie par OPX, SEQ ID N0 : 3,
dans laquelle ledit morphogène stimule la production d'une isoforme de N-CAM ou de L1 via une cellule NG108-15 *in vitro*.

10. Utilisation d'un morphogène pour la préparation d'un médicament destiné à empêcher une dégénérescence des cellules épithéliales pourvues de poils, à l'exclusion d'une régression de la perception sensorielle suite à une ischémie ou à un traumatisme, dans laquelle le morphogène possède une séquence d'acides aminés choisie parmi le groupe de séquences comprenant :
(a) une séquence possédant une homologie à concurrence d'au moins 70 % avec le squelette à extrémité C-terminale comportant sept résidus cystéine de la OP-1 humaine, les résidus 330 - 431 de SEQ ID N0 : 2 ;
(b) une séquence possédant une identité de séquence à concurrence de plus de 60 % avec ledit squelette à extrémité C-terminale comportant sept résidus cystéine de la OP-1 humaine ;
(c) une séquence définie par la Séquence Générique 7, SEQ ID N0 : 4 ;
(d) une séquence définie par la Séquence Générique 8, SEQ ID N0 : 5 ;
(e) une séquence définie par la Séquence Générique 9, SEQ ID N0 : 6 ;
(f) une séquence définie par la Séquence Générique 10, SEQ ID N0 : 7 ; et
(g) une séquence définie par OPX, SEQ ID N0 : 3,
dans laquelle ledit morphogène stimule la production d'une isoforme de N-CAM ou de L1 via une cellule NG108-15 *in vitro*.

11. Utilisation d'un morphogène pour la préparation d'un médicament destiné à favoriser la croissance des cellules épithéliales pourvues de poils), à l'exclusion d'une régression de la perception sensorielle suite à une ischémie ou à un traumatisme, dans laquelle le morphogène possède une séquence d'acides aminés choisie parmi le groupe de séquences comprenant :
(a) une séquence possédant une homologie à concurrence d'au moins 70 % avec le squelette à extrémité C-terminale comportant sept résidus cystéine de la OP-1 humaine, les résidus 330 - 431 de SEQ ID N0 : 2 ;
(b) une séquence possédant une identité de séquence à concurrence de plus de 60 % avec ledit squelette à extrémité C-terminale comportant sept résidus cystéine de la OP-1 humaine ;
(c) une séquence définie par la Séquence Générique 7, SEQ ID N0 : 4 ;
(d) une séquence définie par la Séquence Générique 8, SEQ ID N0 : 5 ;
(e) une séquence définie par la Séquence Générique 9, SEQ ID N0 : 6 ;
(f) une séquence définie par la Séquence Générique 10, SEQ ID N0 : 7 ; et
(g) une séquence définie par OPX, SEQ ID N0 : 3,
dans laquelle ledit morphogène stimule la production d'une isoforme de N-CAM ou de L1 via une cellule NG108-15 *in vitro*.

12. Utilisation selon l'une quelconque des revendications précédentes, dans laquelle ledit morphogène est la OP-1.

13. Utilisation selon l'une quelconque des revendications 1 - 11, dans laquelle ledit morphogène est choisi parmi le groupe constitué par la OP-2 humaine, la OP-2 de souris, 60A, GDF-1, BMP2A, BMP2B, DPP, Vgl, Vgr-1, BMP3, BMP5 et BMP6, ledit morphogène stimulant la production d'une isoforme de N-CAM ou de L1 via une cellule NG108-15 *in vitro*.

14. Utilisation selon l'une quelconque des revendications précédentes, dans laquelle ledit morphogène est soumis à une complexation non covalente avec au moins un domaine pro du morphogène.
